# EUROPEAN PATENT SPECIFICATION

(11) **EP 2 078 531 B1**
(45) Date of publication and mention of the grant of the patent: **30.05.2012**
(21) Application number: 08020754.1
(22) Date of filing: 07.08.2001
(51) Int. Cl.: A61K 39/395

(54) **Pharmaceutical compositions and methods useful for modulating angiogenesis**
Nützliche pharmazeutische Zusammensetzungen und Verfahren für Angiogenesemodulation
Compositions pharmaceutiques et procédés utiles pour la modulation d'angiogénèse

(30) Priority: 08.08.2000 US 223739
(43) Date of publication of application: 15.07.2009
(62) Divisional of application: 01958338.4
(73) Proprietor: TECHNION RESEARCH AND DEVELOPMENT FOUNDATION LTD., Haifa 32 000 (IL)
(72) Inventor: Neufeld, Gera, 34 679 Haifa (IL); Akiri, Gal, 32 884 Haifa (IL); Vadasz, Zhava, 32 446 Haifa (IL); Gengrovitch, Stela, 21 960 Karmiel (IL)
(74) Representative: Schüssler, Andrea

(56) References cited:
- WO-A-00/44910
- WO-A-01/83702
- WO-A-2004/047720
- SAITO H ET AL: "Regulation of a novel gene encoding a lysyl oxidase-related protein in cellular adhesion and senescence" JOURNAL OF BIOLOGICAL CHEMISTRY, AMERICAN SOCIETY OF BIOLOCHEMICAL BIOLOGISTS, BIRMINGHAM, US, vol. 272, no. 13, 28 March 1997 (1997-03-28), pages 8157-8160, XP002187526 ISSN: 0021-9258
- ITO HIROMU ET AL: "Molecular cloning and biological activity of a novel lysyl oxidase-related gene expressed in cartilage" JOURNAL OF BIOLOGICAL CHEMISTRY, AMERICAN SOCIETY OF BIOLOCHEMICAL BIOLOGISTS, BIRMINGHAM, US, vol. 276, no. 26, 29 June 2001 (2001-06-29), pages 24023-24029, XP002199860 ISSN: 0021-9258
- AKIRI GAL ET AL: "Lysyl oxidase-related protein-1 promotes tumor fibrosis and tumor progression in vivo" 20030401, vol. 63, no. 7, 1 April 2003 (2003-04-01), pages 1657-1666, XP002336330
- PEINADO H ET AL: "A molecular role for lysyl oxidase-like 2 enzyme in snail regulation and tumor progression" EMBO JOURNAL, OXFORD UNIVERSITY PRESS, SURREY, GB, vol. 24, no. 19, 5 October 2005 (2005-10-05), pages 3446-3458, XP008091358 ISSN: 0261-4189
- VAN BERGEN ET AL: 'The role of LOXa nd LOXL2 in wound healing after glaucoma filtration surgery', [Online] 08 October 2010, European association vor vision and eye research Retrieved from the Internet: <URL:http://www.ever.be/view_abstract.php?a bs_id=5411>

## Description

### FIELD AND BACKGROUND OF THE INVENTION

The present invention relates to pharmaceutical compositions and methods useful for modulating angiogenesis.

In an adult, formation of new blood vessels in normal or diseased tissues is regulated by two processes, recapitulated vasculogenesis (the transformation of pre-existing arterioles into small muscular arteries) and angiogenesis, the sprouting of existing blood vessels (which occurs both in the embryo and in the adult).

The process of angiogenesis is regulated by biomechanical and biochemical stimuli. Angiogenic factors such as vascular endothelial growth factor (VEGF) and basic fibroblast growth factor (bFGF), are released by vascular cells, macrophages, and cell surrounding blood vessels. These angiogenic factors activate specific proteases which are involved in degradation of the basement membrane. As a result of this degradation, vascular cells migrate and proliferate thus leading to new blood vessel formation. Peri-endothelial cells, such as pericytes in the capillaries, smooth muscle cells in larger vessels and cardiac myocytes in the heart are recruited to provide maintenance and modulatory functions to the forming vessel.

The establishment and remodeling of blood vessels is controlled by paracrine signals, many of which are mediated by protein ligands which modulate the activity of transmembrane tyrosine kinase receptors. Among these molecules are vascular endothelial growth factor (VEGF) and its receptor families (VEGFR-1, VEGFR-2, neuropilin-1 and neuropilin-2), Angiopoietins 1-4 (Ang-1, Ang-2 etc.) and their respective receptors (Tie-1 and Tie-2), basic fibroblast growth factor (bFGF), platelet derived growth factor (PDGF), and transforming growth factor β (TGF-β).

The growth of solid tumors is limited by the availability of nutrients and oxygen. When cells within solid tumors start to produce angiogenic factors or when the levels of angiogenesis inbibitors decline, the balance between anti-angiogenic and angiogenic influences is perturbed, initiating the growth of new blood vessels from the existing vascular bed into the tumor. This event in tumor progression is known as the angiogenic switch (1,2). It had been demonstrated that inhibitors of tumor angiogenesis are able to completely inhibit tumor growth in mice (3,4) and also inhibit tumor metastasis, a process that relies upon close contact between the vasculature and tumor cells (5). It has also been demonstrated that angiogenesis plays an important role in the progression of breast cancer (6-9).

Such finding have prompted the use of known anti-angiogenic factors in breast cancer therapy (10-12) and a search for novel angiogenesis inhibitors.

During the past decade several novel inhibitors of angiogenesis have been isolated including inhibitors of VEGF signaling (13) and inhibitors of processes which lead to the maturation and stabilization of new blood vessels. Anti-integrin antibodies have been used as inhibitors of blood vessel maturation (14,15).

Although several anti-angiogenic drugs are now available commercially, the anti-angiogenic mechanisms of most of these drugs (e.g., angiostatin and endostatin) remain unclear (16,17).

Since angiogenesis can be initiated by many (possibly compansatory) angiogenic factors it stands to reason that anti-angiogenic factors which target later processes in the angiogenic response such as vessel maturation or a combination of anti-angiogenic factors would be most effective in arresting vessel formation.

Platelet factor-4 (PF4) is an anti-angiogenic protein normally sequestered in platelets (18-20). PF4 inhibits angiogenesis using poorly defined mechanisms (21-24). It was previously speculated that PF4 binds to cell surface heparan-sulfate proteoglycans and in this manner inhibits the activity of angiogenic growth factors such as basic fibroblast growth factor (24).

While reducing the present invention to practice and while searching for alternative anti-angiogenic factors or targets, the present inventors have uncovered a novel PF4 binding protein which participates in modulating angiogenesis.

As demonstrated by the present study, this protein, which is termed herein LOR-1, is highly expressed in cultured endothelial cells as well as in other blood vessel cells. In addition, the expression levels of LOR-1 can be correlated to the metastatic properties of breast cancer derived cell lines, indicating that LOR-1 may play additional roles in tumor progression in addition to a role in angiogenesis.

### SUMMARY OF THE INVENTION

According to one aspect of the present invention there is provided a pharmaceutical composition useful for treating a disorder characterized by excessive blood vessel formation, the composition comprising a molecule capable of downregularing a level and/or activity of LOR-1 as defined in the claims.

According to further features of the invention described below, the molecule is an antibody or an antibody fragment capable of binding with, and at least partially inhibiting the activity of, the at least one polypeptide.

According to still further features in the described preferred embodiments the antibody or the antibody fragment is directed against at least a portion of the polypeptide set forth in SEQ ID NOs: 2.

According to still further features in the described preferred embodiments the molecule is a polynucleotide capable of down regulating expression of the at least one type of lysyl-oxidase.

According to still further features in the described preferred embodiments the polynucleotide is at least partially complementary with the polynucleotide set forth in SEQ ID NOs: 1, 4, 5 or 7.

According to still further features in the described preferred embodiments the polypeptide to be downregulated is at least 75% homologous to the polypeptide set forth in SEQ ID NOs: 2.

The present invention successfully addresses the shortcomings of the presently known configurations by providing pharmaceutical compositions and methods which can be used to treat disorders characterized by excessive blood vessel formation.

### BRIEF DESCRIPTION OF THE DRAWINGS

The invention is herein described, by way of example only, with reference to the accompanying drawings. With specific reference now to the drawings in detail, it is stressed that the particulars shown are by way of example and for purposes of illustrative discussion of the preferred embodiments of the present invention only, and are presented in the cause of providing what is believed to be the most useful and readily understood description of the principles and conceptual aspects of the invention. In this regard, no attempt is made to show structural details of the invention in more detail than is necessary for a fundamental understanding of the invention, the description taken with the drawings making apparent to those skilled in the art how the several forms of the invention may be embodied in practice.

In the drawings:

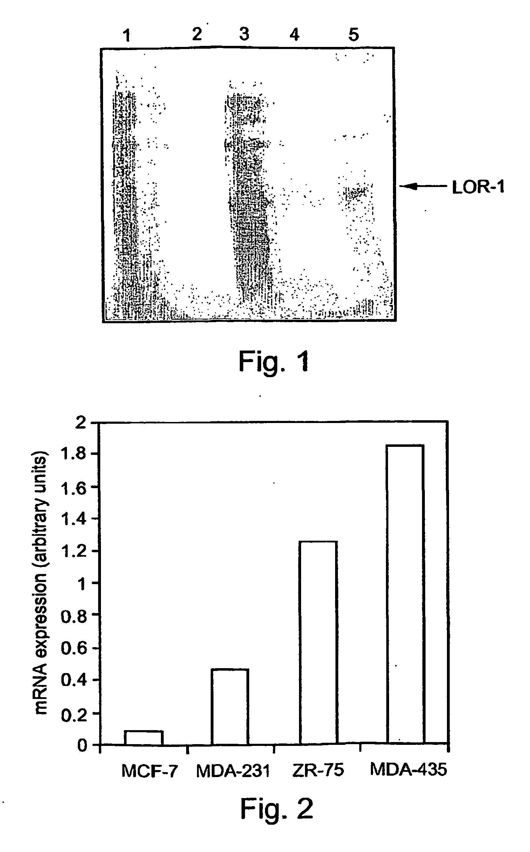

FIG. 1 illustrates SDS-PAGE analysis of extracts of Porcine aortic endothelial cells (PAE Cells) which were transfected with vector along (lane 1) or with vector containing the LOR-1 cDNA (lane 3) and metabolically labeled with ³⁵S-methionine. Extracts from the vector transfected cells (lane 2), from vector containing LOR-1 cDNA transfected cells (lane 4) or from ³⁵S-methionine labeled human umbilical vein endothelial cells (HUVEC) (lane 5) were purified on a PF4 affinity column. A Band corresponding in size to the original band observed in the HUVEC is evident (compare lanes 4 and 5); this band is absent in extracts of vector transfected cells.
FIG. 2 illustrates differential expression of LOR-1 in breast cancer derived cells of different metastatic potential. The metastatic potential of the cells increases from left to right, and is correlated to increased LOR-1 mRNA expression. The results correspond to northern blot analysis of LOR-1 mRNA expression. Data regarding the relative metastatic potential of the cell lines was derived from the literature.

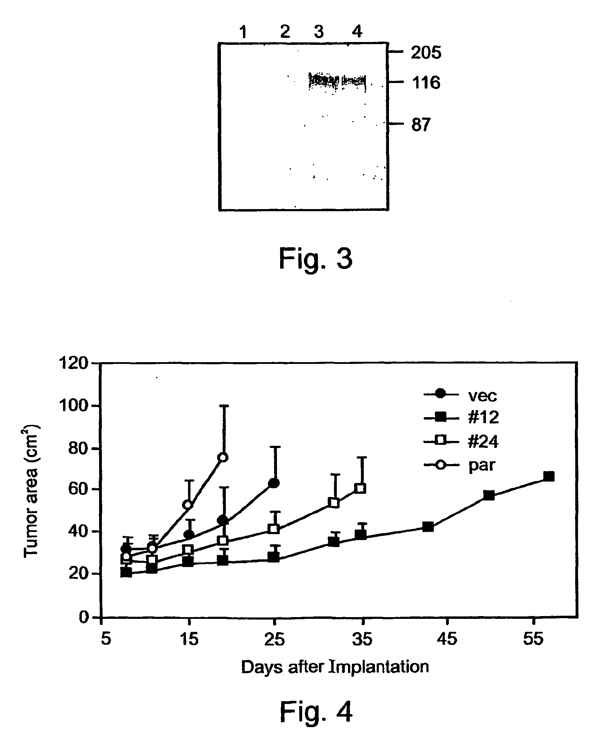

FIG. 3 illustrates expression of recombinant LOR-1 in MCF-7 breast cancer cells (lane 1). Vector transfected MCF-7 cells (lane 2) and two clones of MCF-7 expressing recombinant LOR-1 (lane 3, clone 12, lane 4, clone 22) were grown for two days in serum free medium. The medium from an equal number of cells was collected, concentrated 30 fold using Centricon^{™}, and 10 µl aliquots were electrophoresed using an SDS/PAGE gel. Proteins were blotted onto nitrocellulose, and LOR-1 protein was identified using an antibody directed against the C-terminal of LOR-1. A secondary alkaline-phosphatase coupled antibody and NBT-BICP staining were used to detect the primary bound antibody.
FIG. 4 illustrates tumor size as correlated to LOR-1 expression. Parental MCF-7 cells (par), MCF-7 cells transfected with pCDNA3 vector alone (vec) and two recombinant LOR-1 expressing MCF-7 cells (clones 12 and 24) were deposited under the skin of immune deficient mice (10⁷/injection site) along with an estrogen slow release pellet. Six animals were used for each cell type implanted. The area of the tumors was measured every few days. Bars represent standard deviation from the mean.

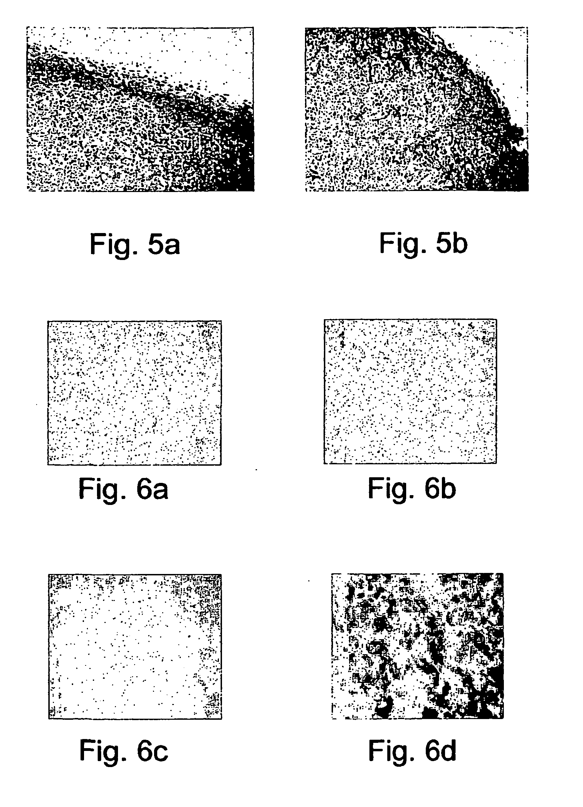

FIGs. 5a-b illustrate anti-factor-8 immunostaining of tumors generated by MCF-7 cells transfected with the expression vector alone (Figure 5a) or with an expression vector containing the LOR-1 cDNA (Figure 5b). Counter staining was performed with hematoxelin-eosin (blue). Invasion of blood vessels into the tumor mass is more abundant in LOR-1 expressing tumors (Figure 5b) as compared to tumors generated by control cells which do not express LOR-1 (Figure 5a).
FIGs. 6a-d illustrate liver sections of Wilson's disease patients (Figures 6c-d) and normal patients (Figures 6a-b) probed with a LOR-1 sense probe (Figures 6a, 6c) and antisense probe (Figures 6b, 6d).

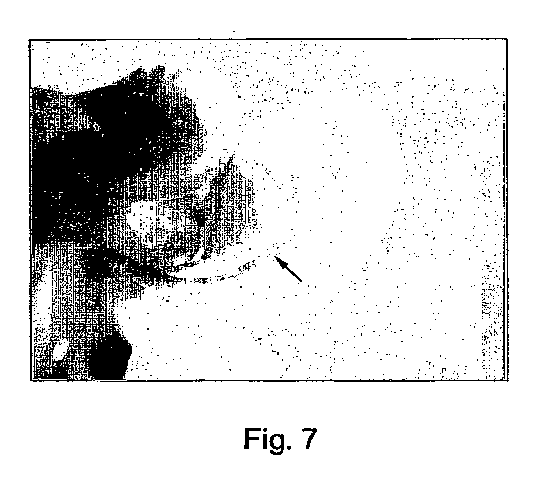

FIG. 7 illustrates results of a whole mount in-situ hybridization of a using a LOR-1 cDNA probe and a 4 day old chick embryo. Strong expression of LOR-1 mRNA is observed in amnionic blood vessels (arrow).

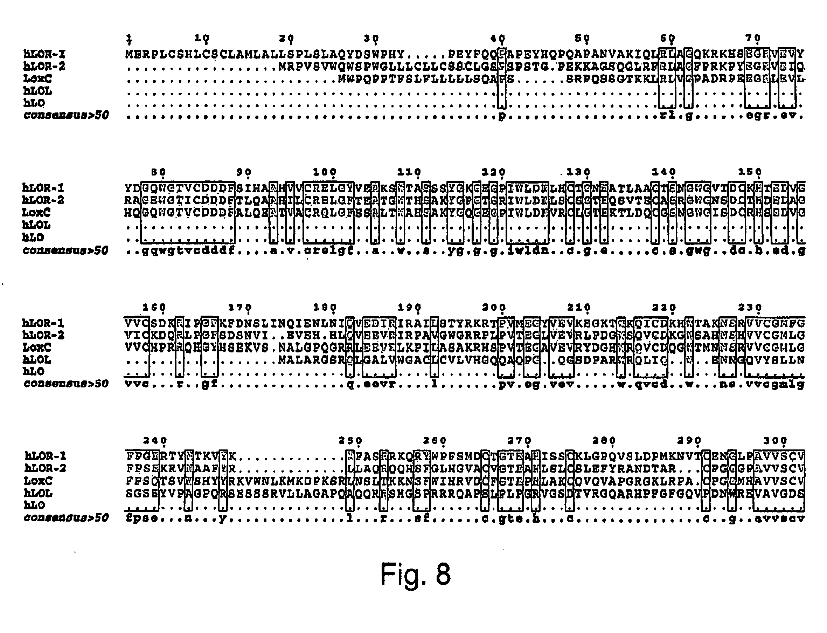

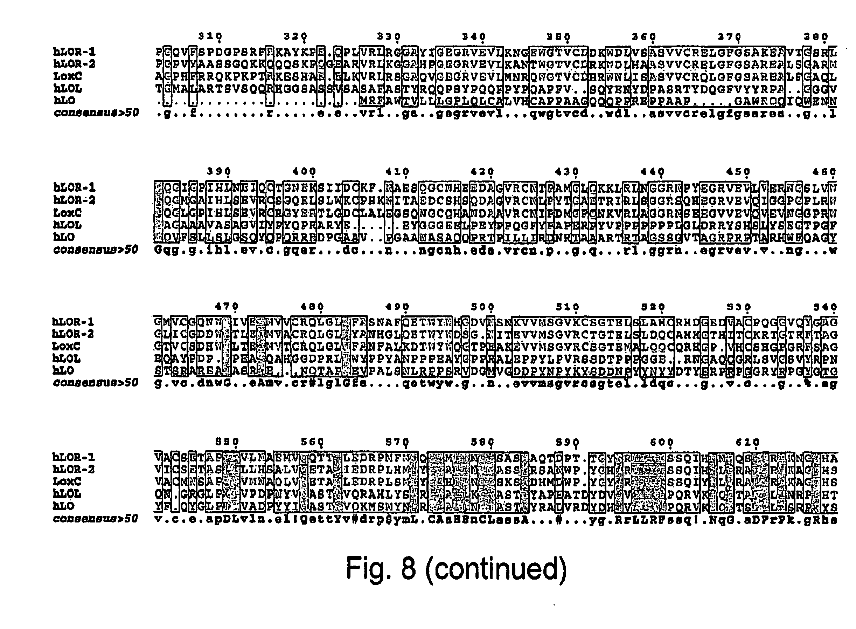

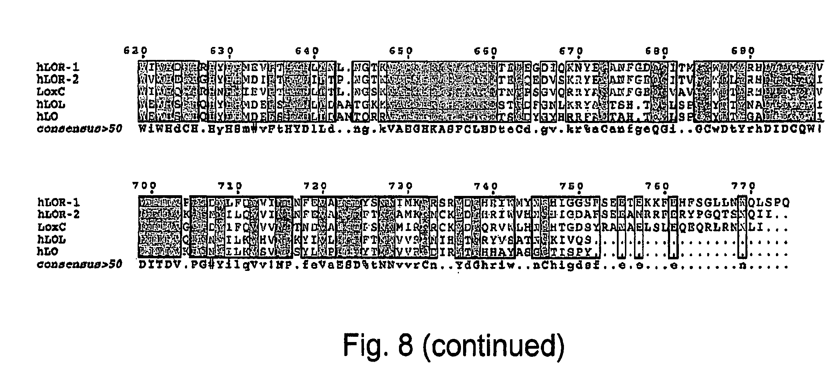

FIG. 8 illustrates sequence alignment of several lysyl oxidases including LOR-1.

### DESCRIPTION OF THE PREFERRED EMBODIMENTS

The present invention is of pharmaceutical compositions and methods which can be used to decrease angiogenesis. Specifically, the present invention can be used to suppress tumor growth and metastasis as well as to treat disorders such as, for example, arthritis, diabetic retinopathy, psoriasis and vasculitis.

The principles and operation of the present invention may be better understood with reference to the drawings and accompanying descriptions.

Before explaining at least one embodiment of the invention in detail, it is to be understood that the invention is not limited in its application to the details of construction and the arrangement of the components set forth in the following description or illustrated in the drawings described in the Examples section. The invention is capable of other embodiments or of being practiced or carried out in various ways. Also, it is to be understood that the phraseology and terminology employed herein is for the purpose of description and should not be regarded as limiting.

As described in the Examples section which, follows, the present inventors have uncovered a novel protein constituent of the angiogenic process.

This protein, which is termed LOR-1 herein (SEQ ID NO:2) belongs to the lysyl-oxidase family of enzymes which catalyze the formation of covalent crosslinks between lysine residues on adjacent collagen or elastin fibrils. The lysyl oxidase family includes four genes (27,28,32,33), the protein sequences of which are presented in SEQ ID NOs: 3, 6, 8 and 9. A homology comparison between several lysyl oxidase family members is presented in Figure 8 which is further described in the Examples section which follows.

Each member of the lysyl-oxidase family of enzymes includes a highly conserved lysyl-oxidase domain, the activity of which is highly dependent on the presence of copper.

It should be noted that prior art studies have shown that removal of copper from tumor tissues leads to inhibition of angiogenesis (30, 34). This further substantiates the role of the lysyl-oxidase family of enzymes in angiogenesis since presumably, removal of copper leads to inhibition of lysyl-oxidases.

Further support to the angiogenic activity of lysyl-oxidases is provided by the PF4-LOR-1 biding assays presented herein. As mentioned hereinabove, PF4 is an inhibitor of angiogenesis. As such, the anti-angiogenic activity exhibited by PF4 may be effected through LOR-1 inhibition, which, as demonstrated in the Examples section which follows, is highly expressed in the endothelial cells lining blood vessels

Thus there is provided a method of modulating angiogenesis.

The method is effected by administering into the mammalian tissue a molecule capable of modifying a tissue level and/or activity of at least one type of lysyl-oxidase to thereby modulate angiogenesis in the mammalian tissue.

As used herein, the phrase "tissue level" refers to the level of lysyl-oxidase protein present in active form in the tissue at a given time point. Protein levels are determined by factors such as, transcription and/or translation rates, RNA or protein turnover and/or protein localization within the cell. As such any molecule which effects any of these factors can modify the tissue level of the lysyl-oxidase.

As used herein the term "activity" refers to an enzymatic activity of the lysyl-oxidase. A molecule which can modify the enzymatic activity may directly or indirectly alter substrate specificity of the enzyme or activity of the catalytic site thereof.

The are numerous examples of molecules which can specifically modify the tissue level and/or activity of a lysyl-oxidase. Such molecules can be categorized into lysyl-oxidase "upregulators" or "downregulators".

### Downregulators

An antibody (polyclonal, monoclonal or monospecific) or an antibody portion (e.g., Fab fragment) directed at least a portion of a lysyl-oxidase (e.g., region spanning the catalytic site) can be used to specifically inhibit lysyl-oxidase activity when introduced into the mammalian tissue, as such, an antibody or an antibody fragment directed at a lysyl-oxidase can be used to suppress or arrest the formation of blood vessels.

Numerous examples of antibody inhibitors are known in the art, including inhibitors of angiogenesis which target angiogenic factors (14, 15).

As is described below, various anti-sense or ribozyme approaches can be used to reduce or abolish transcription or translation of a lysyl oxidase.

An antisense molecule which can be used with the present invention includes a polynucleotide or a polynucleotide analog of at least 10 bases, preferably between 10 and 15, more preferably between 50 and 20 bases, most preferably, at least 17, at least 18, at least 19, at least 20, at least 22, at least 25, at least 30 or at least 40 bases which is hybridizable in vivo, under physiological conditions, with a portion of a polynucleotide strand encoding a polypeptide at least 50 % homologous to SEQ ID NOs:1, 4, 5 or 7 or at least 75 % homologous to an N-terminal portion thereof as determined using the BestFit software of the Wisconsin sequence analysis package, utilizing the Smith and Waterman algorithm, where gap creation penalty equals 8 and gap extension penalty equals 2.

The antisense oligonucleotides used by the present invention can be expressed from nucleic acid construct administered into the tissue, in which case inducible promoters are preferably used such that antisense expression can be switched on and off, or alternatively such oligonucleotides can be chemically synthesized and administered directly into the tissue, as part of, for example; a pharmaceutical composition.

The ability of chemically synthesizing oligonucleotides and analogs thereof having a selected predetermined sequence offers means for downmodulating gene expression. Three types of gene expression modulation strategies may be considered.

At the transcription level, antisense or sense oligonucleotides or analogs that bind to the genomic DNA by strand displacement or the formation of a triple helix, may prevent transcription. At the transcript level, antisense oligonucleotides or analogs that bind target RNA molecules lead to the enzymatic cleavage of the hybrid by intracellular RNase H. In this case, by hybridizing to the targeted mRNA, the oligonucleotides or oligonucleotide analogs provide a duplex hybrid recognized and destroyed by the RNase H enzyme. Alternatively, such hybrid formation may lead to interference with correct splicing. As a result, in both cases, the number of the target mRNA intact transcripts ready for translation is reduced or eliminated. At the translation level, antisense oligonucleotides or analogs that bind target mRNA molecules prevent, by steric hindrance, binding of essential translation factors (ribosomes), to the target mRNA, a phenomenon known in the art as hybridization arrest, disabling the translation of such mRNAs.

Several prior art studies have shown that antisense oligonucleotides can be effective in vivo. For example, antisense molecules have been used to arrest hematopoietic cell proliferation (58), growth (59), or entry into the S phase of the cell cycle (60) and to prevent receptor mediated responses (61).

Several considerations must be taken into account when designing antisense oligonucleotides. For efficient in vivo inhibition of gene expression using antisense oligonucleotides or analogs, the oligonucleotides or analogs must fulfill the following requirements (i) sufficient specificity in binding to the target sequence; (ii) solubility in water; (iii) stability against intra- and extracellular nucleases; (iv) capability of penetration through the cell membrane; and (v) when used to treat an organism, low toxicity.

Unmodified oligonucleotides are typically impractical for use as antisense sequences since they have short in vivo half-lives, during which they are degraded rapidly by nucleases. Furthermore, they are difficult to prepare in more than milligram quantities. In addition, such oligonucleotides are poor cell membrane penetrants.

Thus it is apparent that in order to meet all the above listed requirements, oligonucleotide analogs need to be devised in a suitable manner.

For example, problems arising in connection with double-stranded DNA (dsDNA) recognition through triple helix formation have been diminished by a clever "switch back" chemical linking, whereby a sequence of polypurine on one strand is recognized, and by "switching back", a homopurine sequence on the other strand can be recognized. Also, good helix formation has been obtained by using artificial bases, thereby improving binding conditions with regard to ionic strength and pH.

In addition, in order to improve half-life as well as membrane penetration, a large number of variations in polynucleotide backbones have been done, nevertheless with little success.

Oligonucleotides can be modified either in the base, the sugar or the phosphate moiety. These modifications include, for example, the use of methylphosphonates, monothiophosphates, dithiophosphates, phosphoramidates, phosphate esters, bridged phosphorothioates, bridged phosphoramidates, bridged methylenephosphonates, dephospho internucleotide analogs with siloxane bridges, carbonate bridges, carboxymethyl ester bridges, carbonate bridges, carboxymethyl ester bridges, acetamide bridges, carbamate bridges, thioether bridges, sulfoxy bridges, sulfono bridges, various "plastic" DNAs, anomeric bridges and borane derivatives (62).

International patent application WO 89/12060 discloses various building blocks for synthesizing oligonucleotide analogs, as well as oligonucleotide analogs formed by joining such building blocks in a defined sequence. The building blocks may be either "rigid" (i.e., containing a ring structure) or "flexible" (i.e., lacking a ring structure). In both cases, the building blocks contain a hydroxy group and a mercapto group, through which the building blocks are said to join to form oligonucleotide analogs. The linking moiety in the oligonucleotide analogs is selected from the group consisting of sulfide (-S-), sulfoxide (-SO-), and sulfone (-SO2-).

International patent application WO 92/20702 describe an acyclic oligonucleotide which includes a peptide backbone on which any selected chemical nucleobases or analogs are stringed and serve as coding characters as they do in natural DNA or RNA. These new compounds, known as peptide nucleic acids (PNAs), are not only more stable in cells than their natural counterparts, but also bind natural DNA and RNA 50 to 100 times more tightly than the natural nucleic acids cling to each other. PNA oligomers can be synthesized from the four protected monomers containing thymine, cytosine, adenine and guanine by Merrifield solid-phase peptide synthesis. In order to increase solubility in water and to-prevent aggregation, a lysine amide group is placed at the C-terminal region.

Thus, antisense technology requires pairing of messenger RNA with an oligonucleotide to form a double helix that inhibits translation. The concept of antisense-mediated gene therapy was already introduced in 1978 for cancer therapy. This approach was based on certain genes that are crucial in cell division and growth of cancer cells. Synthetic fragments of genetic substance DNA can achieve this goal. Such molecules bind to the targeted gene molecules in RNA of tumor cells, thereby inhibiting the translation of the genes and resulting in dysfunctional growth of these cells. Other mechanisms has also been proposed. These strategies have been used, with some success in treatment of cancers, as well as other illnesses, including viral and other infectious diseases.

Antisense oligonucleotides are typically synthesized in lengths of 13-30 nucleotides. The life span of oligonucleotide molecules in blood is rather short. Thus, they have to be chemically modified to prevent destruction by ubiquitous nucleases present in the body. Phosphorothioates are very widely used modification in antisense oligonucleotide ongoing clinical trials. A new generation of antisense molecules consist of hybrid antisense oligonucleotide with a central portion of synthetic DNA while four bases on each end have been modified with 2'O-methyl ribose to resemble RNA. In preclinical studies in laboratory animals, such compounds have demonstrated greater stability to metabolism in body tissues and an improved safely profile when compared with the first-generation unmodified phosphorothioate. Dozens of other nucleotide analogs have also been tested in antisense technology.

RNA oligonucleotides may also be used for antisense inhibition as they form a stable RNA-RNA duplex with the target, suggesting efficient inhibition. However, due to their low stability RNA oligonucleotides are typically expressed inside the cells using vectors designed for this purpose. This approach is favored when attempting to target a mRNA_that encodes an abundant and long-lived protein.

RNA oligonucleotides may also be designed so as to activate RNA interference mechanisms within the cell (RNAi). Oligonucleotides suitable for such purpose must be of a defined length and area of complementation (63).

Recent scientific publications have validated the efficacy of antisense compounds in animal models of hepatitis, cancers, coronary artery restenosis and other diseases. The first antisense drug was recently approved by the FDA. The drug, Fomivirsen, was developed by Isis, and is indicated for local treatment of cytomegalovirus in patients with AIDS who are intolerant of or have a contraindication to other treatments for CMV retinitis or who were insufficiently responsive to previous treatments for CMV retinitis (Pharmacotherapy News Network).

Several antisense compounds are now in clinical trials in the United States. These include locally administered antivirals, systemic cancer therapeutics. Antisense therapeutics has the potential to treat many life-threatening diseases with a number of advantages over traditional drugs. Traditional drugs intervene after a disease-causing protein is formed. Antisense therapeutics, however, block mRNA transcription/translation and intervene before a protein is formed, and since antisense therapeutics target only one specific mRNA, they should be more effective with fewer side effects than current protein-inhibiting therapy.

A second option for disrupting gene expression at the level of transcription uses synthetic oligonucleotides capable of hybridizing with double stranded DNA. A triple helix is formed. Such oligonucleotides may prevent binding of transcription factors to the gene's promoter and therefore inhibit transcription. Alternatively, they may prevent duplex unwinding and, therefore, transcription of genes within the triple helical structure.

Ribozymes may also be used as down regulators. Ribozymes are being increasingly used for the sequence specific inhibition -of-gene expression by the cleavage of mRNAs encoding proteins of interest. The possibility of designing ribozymes to cleave any specific target RNA has rendered them valuable tools in both basic research and therapeutic applications. In the therapeutics area, ribozymes have been exploited to target viral RNAs in infectious diseases, dominant oncogenes in cancers and specific somatic mutations in genetic disorders. Most notably, several ribozyme gene therapy protocols for HIV patients are already in Phase 1 trials (67). More recently, ribozymes have been used for transgenic animal research, gene target validation and pathway elucidation. Several ribozymes are in various stages of clinical trials. ANGIOZYME was the first chemically synthesized ribozyme to be studied in human clinical trials. ANGIOZYME specifically inhibits formation of the VEGF-r (Vascular Endothelial Growth Factor receptor), a key component in the angiogenesis pathway. Ribozyme Pharmaceuticals, Inc., as well as other firms have demonstrated the importance of anti-angiogenesis therapeutics in animal models. HEPTAZYME, a ribozyme designed to selectively destroy Hepatitis C Virus (HCV) RNA, was found effective in decreasing Hepatitis C viral RNA in cell culture assays (Ribozyme Pharmaceuticals, Incorporated).

The downregulators described hereinabove would be particularly useful for inhibiting angiogenesis in tumor tissue. It has been shown that PF4, a lysyl oxidase biding protein which inhibits angiogenesis in tumor tissue specifically accumulates in newly formed blood vessels of tumors (angiogenic vessels) but not in established blood vessels (31,35).

Newly formed angiogenic blood vessels are more permeable to proteins than established blood vessels because the major inducer of angiogenesis in many angiogenic diseases is VEGF, a growth factor which also functions as a potent blood vessel permeabilizing factor (VPF) (13). Tumor associated blood vessels are therefore in a permanent state of hyperpermeability due to deregulated over-expression of VEGF (36,37) and as such, a downregulator molecule used by the method of the present invention would be able to extravasate efficiently from tumor blood vessels but much less efficiently from normal stabilized blood vessels.

In order to modulate angiogenesis the downregulator molecules used by the present invention can be administered to the individual per se, or in a pharmaceutical composition where it is mixed with suitable carriers or excipients.

As used herein a "pharmaceutical composition" refers to a preparation of one or more of the active ingredients described herein with other chemical components such as physiologically suitable carries and excipients. The purpose of a pharmaceutical composition is to facilitate administration/targeting of a compound to a mammal.

As used herein the term "active ingredients", refers to the preparation accountable for the biological effect, i.e. the upregulator/downregulator molecules used by the present invention.

Hereinafter, the phrases "physiologically acceptable carrier" and "pharmaceutically acceptable carrier" are interchangeably used to refer to a carrier, such as, for example, a liposome, a virus, a micelle, or a proteins, or a diluent which do not cause significant irritation to the mammal and do not abrogate the biological activity and properties of the active ingredient. An adjuvant is included under these phrases.

Herein the term "excipient" refers to an inert substance added to a pharmaceutical composition to further facilitate administration of an active ingredient. Examples, without limitation, of excipients, include calcium carbonate, calcium phosphate, various sugars and types of starch, cellulose derivatives, gelatin, vegetable oils and polyethylene glycols.

Techniques for formulation and administration of compositions may be found in "Remington's Pharmaceutical Sciences," Mack Publishing Co., Easton, PA, latest edition, which is incorporated herein by reference.

Suitable routes of administration may, for example, include oral, rectal, transmucosal, transnasal, intestinal or parenteral delivery, including intramuscular, subcutaneous and intramedullary injections as well as intrathecal, direct intraventricular, intravenous, inrtaperitoneal, intranasal, or intraocular injections.

For injection, the active ingredients of the invention may be formulated in aqueous solutions, preferably in physiologically compatible buffers such as Hank's solution, Ringer's solution, or physiological salt buffer. For transmucosal administration, penetrants appropriate to the barrier to be permeated are used in the formulation. Such penetrants are generally known in the art.

For oral administration, the compounds can be formulated readily by combining the active ingredient with pharmaceutically acceptable carriers well known in the art. Such carriers enable the active ingredient of the invention to be formulated as tablets, pills, dragees, capsules, liquids, gels, syrups, slurries, suspensions, and the like, for oral ingestion by a patient. Pharmacological preparations for oral use can be made using a solid excipient, optionally grinding the resulting mixture, and processing the mixture of granules, after adding suitable auxiliaries if desired, to obtain tablets or dragee cores. Suitable excipients are, in particular, fillers such as sugars, including lactose, sucrose, mannitol, or sorbitol; cellulose preparations such as, for example, maize starch, wheat starch, rice starch, potato starch, gelatin, gum tragacanth, methyl cellulose, hydroxypropylmethyl-cellulose, sodium carbomethylcellulose; and/or physiologically acceptable polymers such as polyvinylpyrrolidone (PVP). If desired, disintegrating agents may be added, such as cross-linked polyvinyl pyrrolidone, agar, or alginic acid or a salt thereof such as sodium alginate.

Dragee cores are provided with suitable coatings. For this purpose, concentrated sugar solutions may be used which may optionally contain gum arabic, talc, polyvinyl pyrrolidone, carbopol gel, polyethylene glycol, titanium dioxide, lacquer solutions and suitable organic solvents or solvent mixtures. Dyestuffs or pigments may be added to the tablets or dragee coatings for identification or to characterize different combinations of active compound doses.

Pharmaceutical compositions, which can be used orally, include push-fit capsules made of gelatin as well as soft, sealed capsules made of gelatin and a plasticizer, such as glycerol or sorbitol. The push-fit capsules may contain the active ingredients in admixture with filler such as lactose, binders such as starches, lubricants such as talc or magnesium stearate and, optionally, stabilizers. In soft capsules, the active ingredients may be dissolved or suspended in suitable liquids, such as fatty oils, liquid paraffin, or liquid polyethylene glycols. In addition, stabilizers may be added. All formulations for oral administration should be in dosages suitable for the chosen route of administration.

For buccal administration, the compositions may take the form of tablets or lozenges formulated in conventional manner.

The preparations described herein may be formulated for parenteral administration, e.g., by bolus injection or continuos infusion. Formulations for injection may be presented in unit dosage form, e.g., in ampoules or in multidose containers with optionally, an added preservative. The compositions may be suspensions, solutions or emulsions in oily or aqueous vehicles, and may contain formulatory agents such as suspending, stabilizing and/or dispersing agents.

Pharmaceutical compositions for parenteral administration include aqueous solutions of the active preparation in water-soluble form. Additionally, suspensions of the active ingredients may be prepared as appropriate oily or water based injection suspensions. Suitable lipophilic solvents or vehicles include fatty oils such as sesame oil, or synthetic fatty acids esters such as ethyl oleate, triglycerides or liposomes. Aqueous injection suspensions may contain substances, which increase the viscosity of the suspension, such as sodium carboxymethyl cellulose, sorbitol or dextran. Optionally, the suspension may also contain suitable stabilizers or agents which increase the solubility of the active ingredients to allow for the preparation of highly concentrated solutions.

Alternatively, the active ingredient may be in powder form for constitution with a suitable vehicle, e.g., sterile, pyrogen-free water based solution, before use.

The preparation of the present invention may also be formulated in rectal compositions such as suppositories or retention enemas, using, e.g., conventional suppository bases such as cocoa butter or other glycerides.

Pharmaceutical compositions suitable for use in context of the present invention include compositions wherein the active ingredients are contained in an amount effective to achieve the intended purpose.

The pharmaceutical composition may form a part of an article of manufacturing which also includes a packaging material for containing the pharmaceutical composition and a leaflet which provides indications of use for the pharmaceutical composition.

Thus, the present disclosure provides a method and pharmaceutical compositions useful modulating angiogenesis.

Such modulation activity can be used to treat arthritis (38,39), diabetic retinopathy (40), psoriasis (41,42) and vasculitis (43,44).

As such, administration of lysyl oxidase encoding sequences or polypeptides can be used to correct some of the manifestations of these diseases.

In addition, evidence presented in the Examples section which indicates that LOR-1 may be more highly expressed in metastatic cell lines than non-metastatic cell lines (Figure 3) suggests that levels of LOR-1 expression can be used as a diagnostic tool to determine the malignancy of cancer cells, and thus to determine what treatment regimen should be used.

Additional objects, advantages, and novel features of the present invention will become apparent to one ordinarily skilled in the art upon examination of the following examples, which are not intended to be limiting. Additionally, each of the various embodiments and aspects of the present invention as delineated hereinabove and as claimed in the claims section below finds experimental support in the following examples.

### EXAMPLES

Reference is now made to the following examples, which together with the above descriptions, illustrate the invention in a non limiting fashion.

Generally, the nomenclature used herein and the laboratory procedures utilized in the present invention include molecular, biochemical, microbiological and recombinant DNA techniques. Such techniques are thoroughly explained in the literature. See, for example, "Molecular Cloning: A laboratory Manual" Sambrook et al., (1989); "Current Protocols in Molecular Biology" Volumes I-III Ausubel, R. M., ed. (1994); Ausubel et al., "Current Protocols in Molecular Biology", John Wiley and Sons, Baltimore, Maryland (1989); Perbal, "A Practical Guide to Molecular Cloning", John Wiley & Sons, New York (1988); Watson et al., "Recombinant DNA", Scientific American Books, New York; Birren et al. (eds) "Genome Analysis: A Laboratory Manual Series", Vols. 1-4, Cold Spring Harbor Laboratory Press, New York (1998); methodologies as set forth in U.S. Pat. Nos. 4,666,828; 4,683,202; 4,801,531; 5,192,659 and 5,272,057; "Cell Biology: A Laboratory Handbook", Volumes I-III Cellis, J. E., ed. (1994); "Current Protocols in Immunology" Volumes I-III Coligan J. E., ed. (1994); Stites et al. (eds), "Basic and Clinical Immunology" (8th Edition), Appleton & Lange, Norwalk, CT (1994); Mishell and Shiigi (eds), "Selected Methods in Cellular Immunology", W. H. Freeman and Co., New York (1980); available immunoassays are extensively described in the patent and scientific literature, see, for example, U.S. Pat. Nos. 3,791,932; 3,839,153; 3,850,752; 3,850,578; 3,853,987; 3,867,517; 3,879,262; 3,901,654; 3,935,074; 3,984,533; 3,996,345; 4,034,074; 4,098,876; 4,879,219; 5,011,771 and 5,281,521; "Oligonucleotide Synthesis" Gait, M. J., ed. (1984); "Nucleic Acid Hybridization" Hames, B. D., and Higgins S. J., eds. (1985); "Transcription and Translation" Hames, B. D., and Higgins S. J., eds. (1984); "Animal Cell Culture" Freshney, R. I., ed. (1986); "Immobilized Cells and Enzymes" IRL Press, (1986); "A Practical Guide to Molecular Cloning" Perbal, B., (1984) and "Methods in Enzymology" Vol. 1-317, Academic Press; "PCR Protocols: A Guide To Methods And Applications", Academic Press, San Diego, CA (1990); Marshak et al., "Strategies for Protein Purification and Characterization - A Laboratory Course Manual" CSHL Press (1996); all of which are incorporated by reference as if fully set forth herein. Other general references are provided throughout this document. The procedures therein are believed to be well known in the art and are provided for the convenience of the reader.

### EXAMPLE 1

### The role of LOR1 in angiogenesis

A study was conducted in efforts to further substantiate and characterize the role of LOR-1 in angiogenesis.

### Materials and Methods

Human recombinant platelet factor-4 (PF4, GenBank Accession number M20901) which was produced in bacteria and subsequently refolded was supplied by Dr. Maione of Repligen Corp. (Boston, USA). Estrogen slow release pellets were obtained from Innovative Research of America, Sarasota, FL, USA.

***Construction of LOR-1 expression vector, transfection into MCF-7 cells, and expression:*** The LOR-1 cDNA (SEQ ID NO:1) was cloned into a pCDNA3.1-hygro expression vector (Invitrogen Inc., USA) under the control of a CMV promoter. Clones of cells expressing LOR-1 were selected using hygromycine and assayed for LOR-1 expression using the polyclonal antisera described below.

***Construction of platelet factor-4 affinity columns and purification of LOR-1 on such columns:*** PF4 was coupled to sepharose using a modification of the method of Miron and Wilchek (51) as previously described for vascular endothelial growth factor (52). Serum free conditioned medium was collected from ³⁵S-methionine labeled MCF-7 cells which over-expressed LOR-1. The conditioned medium was passed through the column twice. The column was washed with phosphate buffered saline (300 mM NaCl, pH-7.2) and eluted with PBS (containing 2M NaCl).

***Experiments with nude mice:*** Modified or parental MCF-7 cells (10⁷ cells per animal) were implanted under the skin of nude mice. A pellet of slow release estrogen was implanted 1 cm away as previously described (53). Tumors were measured periodically, following which, tumors at least 1 cm in size were removed and immuno-histologicaly analyzed using a commercial antibody directed against a factor 8 like antigen which served as a specific marker for endothelial cells.

***In-situ hybridization:*** Fragments encompassing nucleotides 922-1564 of LOR-1, nucleotides 976-1391 of LOL, nucleotides 400-950 of LO and nucleotides 1061-1590 of LOR-2 (as numbered from the ATG codon of these sequences) where each independently subcloned into the Bluescript SK and KS (Stratagene) vectors. A DIG cRNA labeling kit of Boehringer-Mannheim was used to transcribe sense (s) and antisense (as) digoxigenin-labeled cRNA probes from the T7 promoter of the Bluescript constructs. Hybridization and subsequent detection of hybridized probes was carried out essentially as previously described (54).

***Anti-LOR-1 polyclonal antisera:*** Antisera was generated by injecting a recombinant peptide containing the C-terminal 200 amino-acids of LOR-1 (amino acids 540-744 of SEQ ID NO: 2) into female rabbits. Serum was collected 10 days following each injection and an immunoglobulin fraction was purified using a protein A Sepharose affinity column (Pharmacia).

### Results

***LOR-1 purification:*** A PF4 affinity column was used to detect endothelial cell proteins which specifically interact with PF4.

Two PF4 binding proteins were detected in conditioned medium of human umbilical vein endothelial cells (HUVEC), whereas PF4 binding proteins were not detected in detergent extracts of endothelial cells.

Two liters of conditioned medium enabled a partial purification of one such binding protein which eluted from the column at a relatively high salt concentrations (0.4-0.5 M NaCl).

Further purification of this protein was performed using reverse phase high pressure liquid chromatography and SDS/PAGE chromatography. The PF4 binding protein did not bind to heparin nor was it a heparan-sulfate proteoglycan since heparinase digestion failed to change its mobility in SDS/PAGE experiments.

Partial sequencing and database comparison revealed that the PF4 binding protein of the present invention (LOR-1) belongs to a family of proteins containing a lysyl-oxidase like domain (28,29). Lysyl-oxidases are copper dependent enzymes that participate in the synthesis of the extracellular matrix by catalyzing the formation of covalent bonds between lysines of adjacent collagen or elastin fibers.

The full length amino acid sequence of LOR-1 (as deduced from the isolated cDNA sequence) displayed a high degree of identity to WS9-14, a protein over expressed in senescent fibroblasts, in several types of adherent cells (but not in non-adherent cells) and in fibroblasts, in which it was correlated to pro-collagen I-a1 expression levels, as well as being induced by TGF-β and inhibited by phorbol esters and retinoic acid (27).

***The role of LOR-1 in tumor development:*** Recombinant LOR-1 expressed in PAE cells specifically bound with the PF4 affinity column (Figure 1). Since LOR-1 is a member of the LO family it was hypothesized that it participates in ECM formation during angiogenesis. Furthermore it was also hypothesized that PF4 suppresses or inhibits the pro-angiogenic activity of LOR-1 thus inhibiting the later stages of blood vessel formation and as a result limiting tumor growth.

***LOR-1 expression:*** In-situ hybridization demonstrated that LOR-1 is expressed in a wide variety of tissues and cell types including fibroblasts, adipocytes, nerve cells, endothelial cells and a variety of epithelial cells. Several cell types, such as liver hepatocytes, did not express LOR-1; of the 4 LO family members examined (all except for LoxC) LOR-1 was the only one expressed in endothelial cells of blood vessels.

***LOR-1 and cancer:*** As shown in Figure 2, a direct correlation between the expression levels of LOR-1 and the metastatic properties of breast cancer derived cell lines was demonstrated herein.

Since the epithelial cells which line the milk ducts of normal breast tissue (from which most breast tumors arise) express large amounts of LOR-1 it is possible that the less metastatic lines lost LOR-1 expression rather than gained it.

To substantiate its role in metastasis, LOR-1 cDNA was expressed in non-metastatic breast cancer derived MCF-7 cell lines which do not normally express LOR-1. The expression of LOR-1 was examined using rabbit polyclonal antibodies generated as described above (Figure 3).

A control cell line which was transfected with an empty expression vector, and an MCF-7 cell line expressing LOR-1 were implanted under the skin of immune deficient mice along with an estrogen slow release pellet as described above. Estrogen was added since the development of tumors from this non-metastatic cell line is estrogen dependent (55).

The rate of tumor development in the mice was continuously monitored (Figure 4); tumors 1 cm in size were excised and subjected to histological analysis as described above. Interestingly, the rate of tumor development varied between the two cell lines, with some tumors exhibiting slower growth in the LOR-1 expressing MCF-7 and yet other exhibiting slower growth in the control cells.

In order to overcome expression level problems, LOR-1 cDNA was placed under the control of a tetracycline induced promoter (The TET-off system). Such a construct will enable to determine conclusively whether the reduced rate of tumor growth observed in LOR-1 expressing cells is indeed caused by LOR-1.

Tumors expressing large amounts of LOR-1 were sectioned and stained with an antibody directed against factor 8 like antigen, a specific marker of endothelial cells. Control tumor tissue predominantly stained at the capsule around the tumor while in the LOR-1 expressing tumors pronounced staining was observed in inner regions of the tumor tissue (Figure 5a and Figure 5b respectively).

***The role of LOR-1 in Vilson's disease and in other chronic liver diseases:*** Normal and diseased liver tissue were probed with LOR-1 sense (Figures 6a and 6c) and antisense probes (Figures 6b and 6d). Normal liver tissues expresses very low levels of LOR-1 (Figure 6b). However, fibrotic liver tissues such as those observed in Wilson's disease, exhibit a strong increase in hepatocyte expression of LOR-1 (Figures 6d).

***Expression of LOR-1 in chick embryos:*** Figure 7 illustrates LOR-1 expression of LOR-1 mRNA in blood vessels of a developing chick embryo. Whole-mount in-situ hybridization of 4 day old chick embryos revealed LOR-1 mRNA expression in blood vessels located in the amnion (arrow).

***The Lysyl oxidase family:*** A homology comparison between five members of the lysyl oxidase family which includes the LO and LOL subfamily and the LOR-1 and LOR-2 subfamily revealed a strong homology at the C-terminal portion which includes the conserved lysyl oxidase motif. LOR-1 and LOR-2 are characterized by long N-terminal stretches which are not found in LO and LOL.

### REFERENCES CITED

### (Additional references are cited in the text)

1. Folkman, J. (1990) What is the evidence that tumors are angiogenesis dependent. J. Nat. Cancer Inst. 82, 4-7.
2. Hanahan, D. and Folkman, J. (1996) Patterns and emerging mechanisms of the angiogenic switch during tumorigenesis. Cell 86, 353-364.
3. Boehm., T., Folkman, J., Browder, T., and Oreilly, M. S. (1997) Antiangiogenic therapy of experimental cancer does not induce acquired drug resistance. Nature 390, 404-407.
4. Bergers, G., Javaherian, K., Lo, K. M., Folkman, J., and Hanahan, D. (1999) Effects of angiogenesis inhibitors on multistage carcinogenesis in mice. Science 284, 808-812.
5. Zetter, B. R. (1998) Angiogenesis and tumor metastasis. Annu. Rev. Med. 49:407-424,407-424.
6. Weidner, N. (1998) Tumoural vascularity as a prognostic factor in cancer patients: The evidence continues to grow. J. Pathol. 184, 119-122.
7. Degani, H., Gusis, V., Weinstein, D., Fields, S., and Strano, S. (1997) Mapping pathophysiological features of breast tumors by MRI at high spatial resolution. Nature Med. 3, 780-782.
8. Guidi, A. J., Schnitt, S. J., Fischer, L., Tognazzi, K., Harris, J. R., Dvorak, H. F., and Brown, L. F. (1997) Vascular permeability factor (vascular endothelial growth factor) expression and angiogenesis in patients with ductal carcinoma in situ of the breast. Cancer 80, 1945-1953.
9. Balsari, A., Maier, J. A. M., Colnaghi, M. I., and Menard, S. (1999) Correlation between tumor vascularity, vascular endothelial growth factor production by tumor cells, serum vascular endothelial growth factor levels, and serum angiogenic activity in patients with breast carcinoma. Lab. Invest. 79, 897-902.
10. Klauber, N., Parangi, S., Flynn, E., Hamel, E., and D'Amato, R J. (1997) Inhibition of angiogenesis and breast cancer in mice by the microtubule inhibitors 2-methoxyestradiol and taxol. Cancer Res. 57, 81-86.
11. Harris, A. L., Zhang, H. T., Moghaddam, A., Fox, S., Scott, P., Pattison, A., Gatter, K., Stratford, I., and Bicknell, R. (1996) Breast cancer angiogenesis - New approaches to therapy via antiangiogenesis, hypoxic activated drugs, and vascular targeting. Breast Cancer Res. Treat. 38, 97-108.
12. Weinstatsaslow, D. L., Zabrenetzky, V. S., Vanhoutte, K., Frazier, W. A., Roberts, D. D., and Steeg, P. S. (1994) Transfection of thrombospondin 1 complementary DNA into a human breast carcinoma cell line reduces primary tumor growth, metastatic potential, and angiogenesis. Cancer Res. 54, 6504-6511.
13. Neufeld, G., Cohen, T., Gengrinovitch, S., and Poltorak, Z. (1999) Vascular endothelial growth factor (VEGF) and its receptors. FASEB J. 13, 9-22.
14. Brooks, P. C., Montgomery, A. M. P., Rosenfeld, M., Reisfeld, R. A., Hu, T. H., Klier, G., and Cheresh, D. A. (1994) Integrin alpha(v)beta(3) antagonists promote tumor regression by inducing apoptosis of angiogenic blood vessels. Cell 79, 1157-1164.
15. Brooks, P. C., Silletti, S., Von Schalscha, T. L., Friedlander, M., and Cheresh, D. A. (1998) Disruption of angiogenesis by PEX, a noncatalytic metalloproteinase fragment with integrin binding activity. Cell 92, 391-400.
16. O'Reilly, M. S., Boehm, T., Shing, Y., Fukai, N., Vasios, G., Lane, W. S., Flynn, E., Birkhead, J. R., Olsen, B. R., and Folkman, J. (1997) Endostatin: an endogenous inhibitor of angiogenesis and tumor growth. Cell 88, 277-285.
17. Oreilly, M. S., Holmgren, L., Chen, C., and Folkman, J. (1996) Angiostatin induces and sustains dormancy of human primary tumors in mice. Nature Med. 2, 689-692.
18. Tanaka, T., Manome, Y., Wen, P., Kufe, D. W., and Fine, H. A. (1997) Viral vector-mediated transduction of a modified platelet factor 4 cDNA inhibits angiogenesis and tumor growth. Nature Med. 3, 437-442.
19. Maione, T. E., Gray, G. S., Petro, J., Hunt, A. J., Donner, A. L., Bauer, S. I., Carson, H. F., and Sharpe, R. J. (1990) Inhibition of angiogenesis by recombinant human platelet factor-4 and related peptides. Science 247, 77-79.
20. Neufeld, G., Akiri, G., and Vadasz, Z. (2000) in Platelet Factor 4 (PF4).The Cytokine Reference: A compendium of cytokines and other mediators of host defence (Oppenheim, J. J. and Feldmann, M. eds) Academic Press.
21. Gengrinovitch, S., Greenberg, S. M., Cohen, T., Gitay-Goren, H., Rockwell, P., Maione, T. E., Levi, B., and Neufeld, G. (1995) Platelet factor-4 inhibits the mitogenic activity of VEGF-121 and VEGF-165 using several concurrent mechanisms. J. Biol. Chem. 270, 15059-15065.
22. Brown, K. J. and Parish, C. R. (1994) Histidine-rich glycoprotein and platelet factor 4 mask heparan sulfate proteoglycans recognized by acidic and basic fibroblast growth factor. Biochemistry 33, 13918-13927.
23. Gupta, S. K. and Singh, J. P. (1994) Inhibition of endothelial cell. Proliferation by platelet factor-4 involves a unique action on S phase progression. J. Cell Biol. 127, 1121-1127.
24. Watson, J. B., Getzler, S. B., and Mosher, D. F. (1994) Platelet factor 4 modulates the mitogenic activity of basic fibroblast growth factor. J. Clin. Invest. 94, 261-268.
25. Maione, T. E., Gray, G. S., Hunt, A. J., and Sharpe, R. J. (1991) Inhibition of tumor growth in mice by an analogue of platelet factor 4 that lacks affinity for heparin and retains potent angiostatic activity. Cancer Res. 51, 2077-2083.
26. Sharpe, R. J., Byers, H. R., Scott, C. F., Bauer, S. I., and Maione, T. E. (1990) Growth inhibition of murine melanoma and human colon carcinoma by recombinant human platelet factor 4. J. Natl. Cancer Inst. 82, 848-853.
27. Saito, H., Papaconstantinou, J., Sato, H., and Goldstein, S. (1997) Regulation of a novel gene encoding a lysyl oxidase-related protein in cellular adhesion and senescence. J. Biol. Chem. 272, 8157-8160.
28. Kim, Y., Boyd, C. D., and Csiszar, K. (1995) A new gene with sequence and structural similarity to the gene encoding human lysyl oxidase. J. Biol. Chem. 270, 7176-7182.
29. Kim, Y. H., Peyrol, S., So, C. K., Boyd, C. D., and Csiszar, K. (1999) Coexpression of the lysyl oxidase-like gene (LOXL) and the gene encoding type III procollagen in induced liver fibrosis. J. Cell Biochem. 72, 181-188.
30. Rabinovitz, M. (1999) Angiogenesis and its inhibition: the copper connection. J. Natl. Cancer Inst. 91, 1689-1690.
31. Hansell, P., Maione, T. E., and Borgstrom, P. (1995) Selective binding of platelet factor 4 to regions of active angiogenesis in vivo. Amer. J. Physiol-Heart. Circ. Phy. 38, H829-H836.
32. Reiser, K., McCormick, R. J., and Rucker, R. B. (1992) Enzymatic and nonenzymatic cross-linking of collagen and elastin. FASEB J. 6, 2439-2449.
33. Jang, W., Hua, A., Spilson, S. V., Miller, W., Roe, B. A., and Meisler, M. H. (1999) Comparative sequence of human and mouse BAC clones from the mnd2 region of chromosome 2p13. Genome Res. 9, 53-61.
34. Yoshida, D., Ikeda, Y., and Nakazawa, S. (1995) Copper chelation inhibits tumor angiogenesis in the experimental 9L gliosarcoma model. Neurosurgery 37, 287-292.
35. Borgstroem, P., Discipio, R., and Maione, T. E. (1998) Recombinant platelet factor 4, an angiogenic marker for human breast carcinoma. Anticancer Res. 18, 4035-4041.
36. Shweiki, D., Neeman, M., Itin, A., and Keshet, E. (1995) Induction of vascular endothelial growth factor expression by hypoxia and by glucose deficiency in multicell spheroids: Implications for tumor angiogenesis. Proc. Natl. Acad. Sci. USA 92, 768-772.
37. Rak, J., Mitsuhashi, Y., Bayko, L., Filmus, J., Shirasawa, S., Sasazuki, T., and Kerbel, R. S. (1995) Mutant ras oncogenes upregulate VEGF/VPF expression: Implications for induction and inhibition of tumor angiogenesis. Cancer Res. 55, 4575-4580.
38. Koch, A. E. (1998) Angiogenesis - Implications for rheumatoid arthritis. Arthritis Rheum. 41, 951-962.
39. Paleolog, E. M. and Fava, R. A. (1998) Angiogenesis in rheumatoid arthritis: implications for future therapeutic strategies. Springer Semin. Immunopathol. 20, 73-94.
40. Miller, J. W., Adamis, A. P., and Aiello, L. P. (1997) Vascular endothelial growth factor in ocular neovascularization and proliferative diabetic retinopathy. Diabetes Metab. Rev. 13, 37-50.
41. Detmar, M., Brown, L. F., Claffey, K. P., Yee, K. T., Kocher, O., Jackman, R. W., Berse, B., and Dvorak, H. F. (1994) Overexpression of vascular permeability factor/vascular endothelial growth factor and its receptors in psoriasis. J. Exp. Med. 180, 1141-1146.
42. Creamer, D., Allen, M. H., Sousa, A., Poston, R., and Barker, J. N. W. N. (1997) Localization of endothelial proliferation and microvascular expansion in active plaque psoriasis. Br. J. Dermatol. 136, 859-865.
43. Lie, J. T. (1992) Vasculitis simulators and vasculitis look-alikes. Curr. Opin. Rheumatol. 4,47-55.
44. Klipple, G. L. and Riordan, K. K. (1989) Rare inflammatory and hereditary connective tissue diseases. Rheum. Dis. Clin. North Am. 15, 383-398.
45. Brahn, E., Lehman, T. J. A., Peacock, D. J., Tang, C., and Banquerigo, M. L. (1999) Suppression of coronary vasculitis in a murine model of Kawasaki disease using an angiogenesis inhibitor. Clin. Immunol. Immunopathol. 90, 147-151.
46. Cid, M. C., Grant, D. S., Hoffman, G. S., Auerbach, R., Fauci, A. S., and Kleinman, H. K. (1993) Identification of Haptoglobin as an Angiogenic Factor in Sera from Patients with Systemic Vasculitis. J. Clin. Invest. 91, 977-985.
47. Hoffman, G. S., Filie, J. D., Schumacher, H. R.,Jr., Ortiz-Bravo, E., Tsokos, M. G., Marini, J. C., Kerr, G. S., Ling, Q. H., and Trentham, D. E. (1991) Intractable vasculitis, resorptive osteolysis, and immunity to type I collagen in type VIII Ehlers-Danlos syndrome. Arthritis Rheum. 34, 1466-1475.
48. Bauters, C. and Isner, J. M. (1997) The biology of restenosis. Prog. Cardiovasc. Dis. 40, 107-116.
49. Begelman, S. M. and Olin, J. W. (2000) Fibromuscular dysplasia. Curr. Opin. Rheumatol. 12,41-47.
50. Palta, S., Pai, A. M., Gill, K. S., and Pai, R. G. (2000) New insights into the progression of aortic stenosis : implications for secondary prevention. Circulation 101, 2497-2502.
51. Wilchek, M. Miron, T., 1982. Immobilization of enzymes and affinity ligands onto agarose via stable and uncharged carbamate linkages. Biochem. Int. 4, 629-635.
52. Soker, S., Takashima, S., Miao, H. Q., Neufeld, G., Klagsbrun, M., 1998. Neuropilin-1 is expressed by endothelial and tumor cells as an isoform specific receptor for vascular endothelial growth factor. Cell 92, 735-745.
53. Zhang, H. T., Craft, P., Scott, P. A. E., Ziche, M., Weich, H. A., Harris, A. L., Bicknell, R., 1995. Enhancement of tumor growth and vascular density by transfection of vascular endothelial cell growth factor into MCF-7 human breast carcinoma cells. J. Nat. Cancer Inst. 87, 213-219.
54. Cohen, T., Gluzman-Poltorak, Z., Brodzky, A., Meytal, V., Sabo, E., Misselevich, I., Hassoun, M., Boss, J. H., Resnick, M., Shneyvas, D., Eldar, S., Neufeld, G., 2001. Neuroendocrine Cells along the Digestive Tract Express Neuropilin-2. Biochem. Biophys. Res. Commun. 284, 395-403.
55. Mcleskey, S. W., Kurebayashi, J., Honig, S. F., Zwiebel, J., Lippman, M. E., Dickson, R. B., Kern, F. G., 1993. Fibroblast Growth Factor-4 Transfection of MCF-7 Cells Produces Cell Lines That Are Tumorigenic and Metastatic in Ovariectomized or Tamoxifen-Treated Athymic Nude Mice. Cancer Res. 53, 2168-2177.
56. Nakamura et al.Cancer Res 60(3), 760-5, 2000.
58. Szczylik et al (1991) Selective inhibition of leukemia cell proliferation by BCR-ABL antisense oligodeoxynucleotides. Science 253:562.
59. Calabretta et al. (1991) Normal and leukemic hematopoietic cell manifest differential sensitivity to inhibitory effects of c-myc antisense oligodeoxynucleotides: an in vitro study relevant to bone marrow purging. Proc. Natl. Acad. Sci. USA 88:2351.
60. Heikhila et al. (1987) A c-myc antisense oligodeoxynucleotide inhibits entry into S phase but not progress from G(0) to G(1). Nature, 328:445.
61. Burch and Mahan (1991) Oligodeoxynucleotides antisense to the interleukin I receptor m RNA block the effects of interleukin I in cultured murine and human fibroblasts and in mice. J. Clin. Invest. 88:1190.
62. Cook (1991) Medicinal chemistry of antisense oligonucleotides-future opportunities. Anti-Cancer Drug Design 6:585.
63. Carthew RW. Gene silencing by double-stranded RNA.Curr Opin Cell Biol 2001 Apr;13(2):244-8.
64. S. and Ikeda, J.E.: Trapping of mammalian promoters by Cre-lox site-specific recombination. DNA Res 3 (1996) 73-80.
65. Bedell, M.A., Jenkins, N.A. and Copeland, N.G.: Mouse models of human disease. Part I: Techniques and resources for genetic analysis in mice. Genes and Development 11 (1997) 1-11.
66. Bermingham, J.J., Scherer, S.S., O'Connell, S., Arroyo, E., Kalla, K.A., Powell, F.L. and Rosenfeld, M.G.: Tst-1/Oct-6/SCIP regulates a unique step in peripheral myelination and is required for normal respiration. Genes Dev 10 (1996) 1751-62.
67. Welch P.J., Barber J.R., and Wong-Staal F. (1998) Expression of ribozymes in gene transfer systems to modulate target RNA levels. Curr. Opin. Biotechnol., 9(5):486-496.
68. Bedell-Hogan,D., Trackman,P., Abrams,W., Rosenbloom,J., and Kagan,H. (1993) Oxidation, cross-linking, and insolubilization of recombinant tropoelastin by purified lysyl oxidase. J. Biol. Chem. 268, 10345-10350)

## Claims

1. A pharmaceutical composition for use in a method of treating a disorder **characterized by** excessive blood vessel formation, the composition comprising a molecule capable of down-regulating a level and/or activity of LOR-1, wherein the molecule is selected from:
(a) an antibody or an antibody fragment capable of binding with, and at least partially inhibiting the activity of, LOR-1;
(b) a synthetic oligonucleotide that hybridizes with double-stranded DNA of the LOR-1 gene;
(c) an antisense oligonucleotide or analogue that prevents transcription of LOR-1 mRNA, prevents translation of LOR-1 mRNA, leads to enzymatic cleavage of a hybrid with LOR-1 mRNA, or leads to interference with correct splicing of LOR-1 mRNA;
(d) a ribozyme that inhibits LOR-1 expression by cleavage of LOR-1 mRNA;
(e) an RNA oligonucleotide that activates RNA interference (RNAi) mechanisms, wherein the RNAi mechanisms downregulate LOR-1 expression; and
(f) a nucleic acid construct that expresses the antisense oligonucleotide of (c).

2. The composition of claim 1, wherein said antibody or said antibody fragment is directed against at least an antigenic portion of the polypeptide set forth in SEQ ID NO:2.

3. A pharmaceutical composition for use in a method of treating a disorder **characterized by** excessive blood vessel formation, the composition comprising a molecule capable of down-regulating a level and/or a lysyl oxidase activity of a polypeptide having at least 75% amino acid sequence identity to the polypeptide set forth in SEQ ID NO:2, wherein the molecule is selected from:
(a) an antibody or an antibody fragment capable of binding with, and at least partially inhibiting the activity of, LOR-1;
(b) a synthetic oligonucleotide that hybridizes with double-stranded DNA of the LOR-1 gene;
(c) an antisense oligonucleotide or analogue that prevents transcription of LOR-1 mRNA, prevents translation of LOR-1 mRNA, leads to enzymatic cleavage of a hybrid with LOR-1 mRNA, or leads to interference with correct splicing of LOR-1 mRNA;
(d) a ribozyme that inhibits LOR-1 expression by cleavage of LOR-1 mRNA;
(e) an RNA oligonucleotide that activates RNA interference (RNAi) mechanisms, wherein the RNAi mechanisms downregulate LOR-1 expression; and
(f)a nucleic acid construct that expresses the antisense oligonucleotide of (c).

4. The composition of claim 3, wherein said antibody or said antibody fragment is directed against at least an antigenic portion of the polypeptide set forth in SEQ ID NO:2.

## Patentansprüche

1. Pharmazeutische Zusammensetzung zur Verwendung in einem Verfahren zur Behandlung einer Erkrankung, die durch erhöhte Blutgefäßbildung **gekennzeichnet** ist, wobei die Zusammensetzung ein Molekül umfasst, das das Level und/oder die Aktivität von LOR-1 herunter regulieren kann, wobei das Molekül ausgewählt ist aus:
(a) einem Antikörper oder einem Antikörperfragment, das an LOR-1 binden und seine Aktivität zumindest teilweise hemmen kann;
(b) ein synthetisches Oligonukleotid, das mit der doppelsträngigen DNA des LOR-1-Gens hybridisiert;
(c) ein Antisense-Oligonukleotid oder Analogon, das die Transkription von LOR-1 mRNA verhindert, das die Translation von LOR-1 mRNA verhindert, das zur enzymatischen Spaltung eines Hybrids mit LOR-1 mRNA führt, oder das in das fehlerlose Spleißen von LOR-1 mRNA eingreift;
(d) ein Ribozym, das die LOR-1-Expression durch Spaltung von LOR-1 mRNA hemmt;
(e) ein RNA-Oligonukleotid, das die RNA-Interferenz (RNAi)-Mechanismen aktiviert, wobei die RNAi-Mechanismen die LOR-1-Expression herunter regulieren; und
(f) ein Nukleinsäurekonstrukt, das das Antisense-Oligonukleotid von (c) exprimiert.

2. Die Zusammensetzung nach Anspruch 1, wobei der Antikörper oder das Antikörperfragment gegen mindestens einen antigenen Anteil des Polypeptids gerichtet ist, der in SEQ ID NO: 2 dargelegt ist.

3. Eine pharmazeutische Zusammensetzung zur Verwendung in einem Verfahren zur Behandlung einer Erkrankung, die durch erhöhte Blutgefäßbildung **gekennzeichnet** ist, wobei die Zusammensetzung ein Molekül umfasst, das das Level und/oder die Lysyloxidaseaktivität eines Polypeptids mit mindestens 75% Aminosäureidentität zum Polypeptid, das in SEQ ID NO:2 dargelegt ist, herunter regulieren kann, wobei das Molekül ausgewählt ist aus:
(a) einem Antikörper oder einem Antikörperfragment, das an LOR-1 binden und seine Aktivität zumindest teilweise hemmen kann;
(b) ein synthetisches Oligonukleotid, das mit der doppelsträngigen DNA des LOR-1-Gens hybridisiert;
(c) ein Antisense-Oligonukleotid oder Analogon, das die Transkription von LOR-1 mRNA verhindert, das die Translation von LOR-1 mRNA verhindert, das zur enzymatischen Spaltung eines Hybrids mit LOR-1 mRNA führt, oder das in das fehlerlose Spleißen von LOR-1 mRNA eingreift;
(d) ein Ribozym, das die LOR-1-Expression durch Spaltung von LOR-1 mRNA hemmt;
(e) ein RNA-Oligonukleotid, das die RNA-Interferenz (RNAi)-Mechanismen aktiviert, wobei die RNAi-Mechanismen die LOR-1-Expression herunter regulieren; und
(f) ein Nukleinsäurekonstrukt, das das Antisense-Oligonukleotid von (c) exprimiert.

4. Die Zusammensetzung nach Anspruch 3, wobei der Antikörper oder das Antikörperfragment gegen mindestens einen antigenen Anteil des Polypeptids gerichtet ist, der in SEQ ID NO: 2 dargelegt ist.

## Revendications

1. Composition pharmaceutique destinée à être utilisée pour un procédé de traitement d'un trouble **caractérisé par** une formation excessive de vaisseaux sanguins, la composition comprenant une molécule capable de réguler de façon négative un niveau et/ou une activité du LOR-1, cette molécule étant sélectionnée parmi :
a) un anticorps ou un fragment d'anticorps capable de se lier avec le, et d'inhiber au moins partiellement l'activité du, LOR-1 ;
b) un oligonucléotide synthétique qui s'hybride avec l'ADN bicaténaire du gène LOR-1 ;
c) un oligonucléotide ou analogue antisens qui prévient la transcription du mARN du LOR-1, prévient la traduction du mARN du LOR-1, mène à un clivage enzymatique d'un hybride avec le mARN du LOR-1 ou mène à une interférence avec l'épissage correct du mARN du LOR-1 ;
d) un ribozyme qui inhibe l'expression du LOR-1 par un clivage du mARN du LOR-1 ;
e) un oligonucléotide d'ARN qui active les mécanismes d'interférence de l'ARN (RNAi), ces mécanismes RNAi régulant de façon négative l'expression du LOR-1 ; et
f) une construction d'acide nucléique qui exprime l'oligonucléotide antisens de c).

2. Composition selon la revendication 1, dans laquelle ledit anticorps ou ledit fragment d'anticorps est dirigé contre au moins une partie antigénique du polypeptide présenté dans la séquence SEQ ID n° 2.

3. Composition pharmaceutique destinée à être utilisée pour un procédé de traitement d'un trouble **caractérisé par** une formation excessive de vaisseaux sanguins, la composition comprenant une molécule capable de réguler de façon négative un niveau et/ou une activité de lysyl-oxydase d'un polypeptide ayant une identité de séquence acide aminé d'au moins 75 % avec le polypeptide présenté dans la séquence SEQ ID n° 2, cette molécule étant sélectionnée parmi :
a) un anticorps ou un fragment d'anticorps capable de se lier avec le, et d'inhiber au moins partiellement l'activité du, LOR-1 ;
b) un oligonucléotide synthétique qui s'hybride avec l'ADN bicaténaire du gène LOR-1 ;
c) un oligonucléotide ou analogue antisens qui prévient la transcription du mARN du LOR-1, prévient la traduction du mARN du LOR-1, mène à un clivage enzymatique d'un hybride avec le mARN du LOR-1 ou mène à une interférence avec l'épissage correct du mARN du LOR-1 ;
d) un ribozyme qui inhibe l'expression du LOR-1 par un clivage du mARN du LOR-1 ;
e) un oligonucléotide d'ARN qui active les mécanismes d'interférence de l'ARN (RNAi), ces mécanismes RNAi régulant de façon négative l'expression du LOR-1 ; et
f) une construction d'acide nucléique qui exprime l'oligonucléotide antisens de c).

4. Composition selon la revendication 3, dans laquelle ledit anticorps ou ledit fragment d'anticorps est dirigé contre au moins une partie antigénique du polypeptide présenté dans la séquence SEQ ID n° 2.


### SEQUENCE LISTING

<110> Neufeld, Gera
   Gengrinovitch, Stela
   akiri , Gal
   Vadaz, Zehava
<120> PHARMACEUTICAL COMPOSITIONS AND METHODS USEFUL FOR MODULATING ANGIOGENESIS
<130> 01/22064
<150> US 60/223,739
   <151> 2000-08-08
<160> 9
<170> PatentIn version 3.1
<210> 1
   <211> 2325
   <212> DNA
   <213> Homo sapiens
<400> 1
<210> 2
   <211> 774
   <212> PRT
   <213> Homo sapiens
<400> 2
   Met Glu Arg Pro Leu Cys Ser His Leu Cys Ser Cys Leu Ala Met Leu
   1               5                   10                  15
   Ala Leu Leu Ser Pro Leu Ser Leu Ala Gln Tyr Asp Ser Trp Pro His
               20                  25                  30
   Tyr Pro Glu Tyr Phe Gln Gln Pro Ala Pro Glu Tyr His Gln Pro Gln
           35                  40                  45
   Ala Pro Ala Asn Val Ala Lys Ile Gln Leu Arg Leu Ala Gly Gln Lys
       50                  55                  60
   Arg Lys His Ser Glu Gly Arg Val Glu Val Tyr Tyr Asp Gly Gln Trp
   65                  70                  75                  80
   Gly Thr Val Cys Asp Asp Asp Phe Ser Ile His Ala Ala His Val Val
                   85                  90                  95
   Cys Arg Glu Leu Gly Tyr Val Glu Ala Lys Ser Trp Thr Ala Ser Ser
               100                 105                 110
   Ser Tyr Gly Lys Gly Glu Gly Pro Ile Trp Leu Asp Asn Leu His Cys
           115                 120                 125
   Thr Gly Asn Glu Ala Thr Leu Ala Ala Cys Thr Ser Asn Gly Trp Gly
       130                 135                 140
   Val Thr Asp Cys Lys His Thr Glu Asp Val Gly Val Val Cys Ser Asp
   145                 150                 155                 160
   Lys Arg Ile Pro Gly Phe Lys Phe Asp Asn Ser Leu Ile Asn Gln Ile
                   165                 170                 175
   Glu Asn Leu Asn Ile Gln Val Glu Asp Ile Arg Ile Arg Ala Ile Leu
               180                 185                 190
   Ser Thr Tyr Arg Lys Arg Thr Pro Val Met Glu Gly Tyr Val Glu Val
           195                 200                 205
   Lys Glu Gly Lys Thr Trp Lys Gln Ile Cys Asp Lys His Trp Thr Ala
       210                 215                 220
   Lys Asn Ser Arg Val Val Cys Gly Met Phe Gly Phe Pro Gly Glu Arg
   225 230 235 240
   Thr Tyr Asn Thr Lys Val Tyr Lys Met Phe Ala Ser Arg Arg Lys Gln
   245 250 255
   Arg Tyr Trp Pro Phe Ser Met Asp Cys Thr Gly Thr Glu Ala His Ile
   260 265 270
   Ser Ser Cys Lys Leu Gly Pro Gln Val Ser Leu Asp Pro Met Lys Asn
   275 280 285
   Val Thr Cys Glu Asn Gly Leu Pro Ala Val Val Ser Cys Val Pro Gly
   290 295 300
   Gln Val Phe Ser Pro Asp Gly Pro Ser Arg Phe Arg Lys Ala Tyr Lys
   305 310 315 320
   Pro Glu Gln Pro Leu Val Arg Leu Arg Gly Gly Ala Tyr Ile Gly Glu
   325 330 335
   Gly Arg Val Glu Val Leu Lys Asn Gly Glu Trp Gly Thr Val Cys Asp
   340 345 350
   Asp Lys Trp Asp Leu Val Ser Ala Ser Val Val Cys Arg Glu Leu Gly
   355 360 365
   Phe Gly Ser Ala Lys Glu Ala Val Thr Gly Ser Arg Leu Gly Gln Gly
   370 375 380
   Ile Gly Pro Ile His Leu Asn Glu Ile Gln Cys Thr Gly Asn Glu Lys
   385 390 395 400
   Ser Ile Ile Asp Cys Lys Phe Asn Ala Glu Ser Gln Gly Cys Asn His
   405 410 415
   Glu Glu Asp Ala Gly Val Arg Cys Asn Thr Pro Ala Met Gly Leu Gln
   420 425 430
   Lys Lys Leu Arg Leu Asn Gly Gly Arg Asn Pro Tyr Glu Gly Arg Val
   435 440 445
   Glu Val Leu Val Glu Arg Asn Gly Ser Leu Val Trp Gly Met Val Cys
   450 455 460
   Gly Gln Asn Trp Gly Ile Val Glu Ala Met Val Val Cys Arg Gln Leu
   465 470 475 480
   Gly Leu Gly Phe Ala Ser Asn Ala Phe Gln Glu Thr Trp Tyr Trp His
   485 490 495
   Gly Asp Val Asn Ser Asn Lys Val Val Met Ser Gly Val Lys Cys Ser
   500 505 510
   Gly Thr Glu Leu Ser Leu Ala His Cys Arg His Asp Gly Glu Asp Val
   515 520 525
   Ala Cys Pro Gln Gly Gly Val Gln Tyr Gly Ala Gly Val Ala Cys Ser
   530 535 540
   Glu Thr Ala Pro Asp Leu Val Leu Asn Ala Glu Met Val Gln Gln Thr
   545 550 555 560
   Thr Tyr Leu Glu Asp Arg Pro Met Phe Met Leu Gln Cys Ala Met Glu
   565 570 575
   Glu Asn Cys Leu Ser Ala Ser Ala Ala Gln Thr Asp Pro Thr Thr Gly
   580 585 590
   Tyr Arg Arg Leu Leu Arg Phe Ser Ser Gln Ile His Asn Asn Gly Gln
   595 600 605
   Ser Asp Phe Arg Pro Lys Asn Gly Arg His Ala Trp Ile Trp His Asp
   610 615 620
   Cys His Arg His Tyr His Ser Met Glu Val Phe Thr His Tyr Asp Leu
   625 630 635 640
   Leu Asn Leu Asn Gly Thr Lys Val Ala Glu Gly His Lys Ala Ser Phe
   645 650 655
   Cys Leu Glu Asp Thr Glu Cys Glu Gly Asp Ile Gln Lys Asn Tyr Glu
   660 665 670
   Cys Ala Asn Phe Gly Asp Gln Gly Ile Thr Met Gly Cys Trp Asp Met
   675 680 685
   Tyr Arg His Asp Ile Asp Cys Gln Trp Val Asp Ile Thr Asp Val Pro
   690 695 700
   Pro Gly Asp Tyr Leu Phe Gln Val Val Ile Asn Pro Asn Phe Glu Val
   705 710 715 720
   Ala Glu Ser Asp Tyr Ser Asn Asn Ile Met Lys Cys Arg Ser Arg Tyr
   725 730 735
   Asp Gly His Arg Ile Trp Met Tyr Asn Cys His Ile Gly Gly Ser Phe
   740 745 750
   Ser Glu Glu Thr Glu Lys Lys Phe Glu His Phe Ser Gly Leu Leu Asn
   755 760 765
   Asn Gln Leu Ser Pro Gln
   770
<210> 3
   <211> 757
   <212> PRT
   <213> Homo sapiens
<400> 3
   Met Met Trp Pro Gln Pro Pro Thr Phe Ser Leu Phe Leu Leu Leu Leu
   1 5 10 15
   Leu Ser Gln Ala Pro Ser Ser Arg Pro Gln Ser Ser Gly Thr Lys Lys
   20 25 30
   Leu Arg Leu Val Gly Pro Ala Asp Arg Pro Glu Glu Gly Arg Leu Glu
   35 40 45
   Val Leu His Gln Gly Gln Trp Gly Thr Val Cys Asp Asp Asp Phe Ala
   50 55 60
   Leu Gln Glu Ala Thr Val Ala Cys Arg Gln Leu Gly Phe Glu Ser Ala
   65 70 75 80
   Leu Thr Trp Ala His Ser Ala Lys Tyr Gly Gln Gly Glu Gly Pro Ile
   85 90 95
   Trp Leu Asp Asn Val Arg Cys Leu Gly Thr Glu Lys Thr Leu Asp Gln
   100 105 110
   Cys Gly Ser Asn Gly Trp Gly Ile Ser Asp Cys Arg His Ser Glu Asp
   115 120 125
   Val Gly Val Val Cys His Pro Arg Arg Gln His Gly Tyr His Ser Glu
   130 135 140
   Lys Val Ser Asn Ala Leu Gly Pro Gln Gly Arg Arg Leu Glu Glu Val
   145 150 155 160
   Arg Leu Lys Pro Ile Leu Ala Ser Ala Lys Arg His Ser Pro Val Thr
   165 170 175
   Glu Gly Ala Val Glu Val Arg Tyr Asp Gly His Trp Arg Gln Val Cys
               180                 185                 190
   Asp Gln Gly Trp Thr Met Asn Asn Ser Arg Val Val Cys Gly Met Leu
           195                 200                 205
   Gly Phe Pro Ser Gln Thr Ser Val Asn Ser His Tyr Tyr Arg Lys Val
       210                 215                 220
   Trp Asn Leu Lys Met Lys Asp Pro Lys Ser Arg Leu Asn Ser Leu Thr
   225                 230                 235                 240
   Lys Lys Asn Ser Phe Trp Ile His Arg Val Asp Cys Phe Gly Thr Glu
                   245                 250                 255
   Pro His Leu Ala Lys Cys Gln Val Gln Val Ala Pro Gly Arg Gly Lys
               260                 265                 270
   Leu Arg Pro Ala Cys Pro Gly Gly Met His Ala Val Val Ser Cys Val
           275                 280                 285
   Ala Gly Pro His Phe Arg Arg Gln Lys Pro Lys Pro Thr Arg Lys Glu
       290                 295                 300
   Ser His Ala Glu Glu Leu Lys Val Arg Leu Arg Ser Gly Ala Gln Val
   305                 310                 315                 320
   Gly Glu Gly Arg Val Glu Val Leu Met Asn Arg Gln Trp Gly Thr Val
                   325                 330                 335
   Cys Asp His Arg Trp Asn Leu Ile Ser Ala Ser Val Val Cys Arg Gln
               340                 345                 350
   Leu Gly Phe Gly Ser Ala Arg Glu Ala Leu Phe Gly Ala Gln Leu Gly
           355                 360                 365
   Gln Gly Leu Gly Pro Ile His Leu Ser Glu Val Arg Cys Arg Gly Tyr
       370                 375                 380
   Glu Arg Thr Leu Gly Asp Cys Leu Ala Leu Glu Gly Ser Gln Asn Gly
   385                 390                 395                 400
   Cys Gln His Ala Asn Asp Ala Ala Val Arg Cys Asn Ile Pro Asp Met
                   405                 410                 415
   Gly Phe Gln Asn Lys Val Arg Leu Ala Gly Gly Arg Asn Ser Glu Glu
               420                 425                 430
   Gly Val Val Glu Val Gln Val Glu Val Asn Gly Gly Pro Arg Trp Gly
           435                 440                 445
   Thr Val Cys Ser Asp His Trp Gly Leu Thr Glu Ala Met Val Thr Cys
       450                 455                 460
   Arg Gln Leu Gly Leu Gly Phe Ala Asn Phe Ala Leu Lys Asp Thr Trp
   465                 470                 475                 480
   Tyr Trp Gln Gly Thr Pro Glu Ala Lys Glu Val Val Met Ser Gly Val
                   485                 490                 495
   Arg Cys Ser Gly Thr Glu Met Ala Leu Gln Gln Cys Gln Arg His Gly
               500                 505                 510
   Pro Val His Cys Ser His Gly Pro Gly Arg Phe Ser Ala Gly Val Ala
           515                 520                 525
   Cys Met Asn Ser Ala Pro Asp Leu Val Met Asn Ala Gln Leu Val Gln
       530                 535                 540
   Glu Thr Ala Tyr Leu Glu Asp Arg Pro Leu Ser Met Leu Tyr Cys Ala
   545                 550                 555                 560
   His Glu Glu Asn Cys Leu Ser Lys Ser Ala Asp His Met Asp Trp Pro
                   565                 570                 575
   Tyr Gly Tyr Arg Arg Leu Leu Arg Phe Ser Ser Gln Ile Tyr Asn Leu
               580                 585                 590
   Gly Arg Ala Asp Phe Arg Pro Lys Ala Gly Arg His Ser Trp Ile Trp
           595                 600                 605
   His Gln Cys His Arg His Asn His Ser Ile Glu Val Phe Thr His Tyr
       610                 615                 620
   Asp Leu Leu Thr Leu Asn Gly Ser Lys Val Ala Glu Gly His Lys Ala
   625                 630                 635                 640
   Ser Phe Cys Leu Glu Asp Thr Asn Cys Pro Ser Gly Val Gln Arg Arg
                   645                 650                 655
   Tyr Ala Cys Ala Asn Phe Gly Glu Gln Gly Val Ala Val Gly Cys Trp
               660                 665                 670
   Asp Thr Tyr Arg His Asp Ile Asp Cys Gln Trp Val Asp Ile Thr Asp
           675                 680                 685
   Val Gly Pro Gly Asp Tyr Ile Phe Gln Val Val Val Asn Pro Thr Asn
   690 695 700
   Asp Val Ala Glu Ser Asp Phe Ser Asn Asn Met Ile Arg Cys Arg Cys
   705 710 715 720
   Lys Tyr Asp Gly Gln Arg Val Trp Leu His Asn Cys His Thr Gly Asp
   725 730 735
   Ser Tyr Arg Ala Asn Ala Glu Leu Ser Leu Glu Gln Glu Gln Arg Leu
   740 745 750
   Arg Asn Asn Leu Ile
   755
<210> 4
   <211> 2262
   <212> DNA
   <213> Homo sapiens
<400> 4
<210> 5
   <211> 1725
   <212> DNA
   <213> Homo sapiens
<400> 5
<210> 6
   <211> 574
   <212> PRT
   <213> Homo sapiens
<400> 6
   Met Ala Leu Ala Arg Gly Ser Arg Gln Leu Gly Ala Leu Val Trp Gly
   1               5                   10                  15
   Ala Cys Leu Cys Val Leu Val His Gly Gln Gln Ala Gln Pro Gly Gln
               20                  25                  30
   Gly Ser Asp Pro Ala Arg Trp Arg Gln Leu Ile Gln Trp Glu Asn Asn
   35 40 45
   Gly Gln Val Tyr Ser Leu Leu Asn Ser Gly Ser Glu Tyr Val Pro Ala
   50 55 60
   Gly Pro Gln Arg Ser Glu Ser Ser Ser Arg Val Leu Leu Ala Gly Ala
   65 70 75 80
   Pro Gln Ala Gln Gln Arg Arg Ser His Gly Ser Pro Arg Arg Arg Gln
   85 90 95
   Ala Pro Ser Leu Pro Leu Pro Gly Arg Val Gly Ser Asp Thr Val Arg
   100 105 110
   Gly Gln Ala Arg His Pro Phe Gly Phe Gly Gln Val Pro Asp Asn Trp
   115 120 125
   Arg Glu Val Ala Val Gly Asp Ser Thr Gly Met Ala Leu Ala Arg Thr
   130 135 140
   Ser Val Ser Gln Gln Arg His Gly Gly Ser Ala Ser Ser Val Ser Ala
   145 150 155 160
   Ser Ala Phe Ala Ser Thr Tyr Arg Gln Gln Pro Ser Tyr Pro Gln Gln
   165 170 175
   Phe Pro Tyr Pro Gln Ala Pro Phe Val Ser Gln Tyr Glu Asn Tyr Asp
   180 185 190
   Pro Ala Ser Arg Thr Tyr Asp Gln Gly Phe Val Tyr Tyr Arg Pro Ala
   195 200 205
   Gly Gly Gly Val Gly Ala Gly Ala Ala Ala Val Ala Ser Ala Gly Val
   210 215 220
   Ile Tyr Pro Tyr Gln Pro Arg Ala Arg Tyr Glu Glu Tyr Gly Gly Gly
   225 230 235 240
   Glu Glu Leu Pro Glu Tyr Pro Pro Gln Gly Phe Tyr Pro Ala Pro Glu
   245 250 255
   Arg Pro Tyr Val Pro Pro Pro Pro Pro Pro Pro Asp Gly Leu Asp Arg
   260 265 270
   Arg Tyr Ser His Ser Leu Tyr Ser Glu Gly Thr Pro Gly Phe Glu Gln
   275 280 285
   Ala Tyr Pro Asp Pro Gly Pro Glu Ala Ala Gln Ala His Gly Gly Asp
   290 295 300
   Pro Arg Leu Gly Trp Tyr Pro Pro Tyr Ala Asn Pro Pro Pro Glu Ala
   305 310 315 320
   Tyr Gly Pro Pro Arg Ala Leu Glu Pro Pro Tyr Leu Pro Val Arg Ser
   325 330 335
   Ser Asp Thr Pro Pro Pro Gly Gly Glu Arg Asn Gly Ala Gln Gln Gly
   340 345 350
   Arg Leu Ser Val Gly Ser Val Tyr Arg Pro Asn Gln Asn Gly Arg Gly
   355 360 365
   Leu Pro Asp Leu Val Pro Asp Pro Asn Tyr Val Gln Ala Ser Thr Tyr
   370 375 380
   Val Gln Arg Ala His Leu Tyr Ser Leu Arg Cys Ala Ala Glu Glu Lys
   385 390 395 400
   Cys Leu Ala Ser Thr Ala Tyr Ala Pro Glu Ala Thr Asp Tyr Asp Val
   405 410 415
   Arg Val Leu Leu Arg Phe Pro Gln Arg Val Lys Asn Gln Gly Thr Ala
   420 425 430
   Asp Phe Leu Pro Asn Arg Pro Arg His Thr Trp Glu Trp His Ser Cys
   435 440 445
   His Gln His Tyr His Ser Met Asp Glu Phe Ser His Tyr Asp Leu Leu
   450 455 460
   Asp Ala Ala Thr Gly Lys Lys Val Ala Glu Gly His Lys Ala Ser Phe
   465 470 475 480
   Cys Leu Glu Asp Ser Thr Cys Asp Phe Gly Asn Leu Lys Arg Tyr Ala
   485 490 495
   Cys Thr Ser His Thr Gln Gly Leu Ser Pro Gly Cys Tyr Asp Thr Tyr
   500 505 510
   Asn Ala Asp Ile Asp Cys Gln Trp Ile Asp Ile Thr Asp Val Gln Pro
   515 520 525
   Gly Asn Tyr Ile Leu Lys Val His Val Asn Pro Lys Tyr Ile Val Leu
   530 535 540
   Glu Ser Asp Phe Thr Asn Asn Val Val Arg Cys Asn Ile His Tyr Thr
   545 550 555 560
   Gly Arg Tyr Val Ser Ala Thr Asn Cys Lys Ile Val Gln Ser
   565 570
<210> 7
   <211> 1254
   <212> DNA
   <213> Homo sapiens
<400> 7
   atgcgcttcg cctggaccgt gctcctgctc gggcctttgc agctctgcgc gctagtgcac 60
   tgcgcccctc ccgccgccgg ccaacagcag cccccgcgcg agccgccggc ggctccgggc 120
   gcctggcgcc agcagatcca atgggagaac aacgggcagg tgttcagctt gctgagcctg 180
   ggctcacagt accagcctca gcgccgccgg gacccgggcg ccgccgtccc tggtgcagcc 240
   aacgcctccg cccagcagcc ccgcactccg atcctgctga tccgcgacaa ccgcaccgcc 300
   gcggcgcgaa cgcggacggc cggctcatct ggagtcaccg ctggccgccc caggcccacc 360
   gcccgtcact ggttccaagc tggctactcg acatctagag cccgcgaagc tggcgcctcg 420
   cgcgcggaga accagacagc gccgggagaa gttcctgcgc tcagtaacct gcggccgccc 480
   agccgcgtgg acggcatggt gggcgacgac ccttacaacc cctacaagta ctctgacgac 540
   aacccttatt acaactacta cgatacttat gaaaggccca gacctggggg caggtaccgg 600
   cccggatacg gcactggcta cttccagtac ggtctcccag acctggtggc cgacccctac 660
   tacatccagg cgtccacgta cgtgcagaag atgtccatgt acaacctgag atgcgcggcg 720
   gaggaaaact gtctggccag tacagcatac agggcagatg tcagagatta tgatcacagg 780
   gtgctgctca gatttcccca aagagtgaaa aaccaaggga catcagattt cttacccagc 840
   cgaccaagat attcctggga atggcacagt tgtcatcaac attaccacag tatggatgag 900
   tttagccact atgacctgct tgatgccaac acccagagga gagtggctga aggccacaaa 960
   gcaagtttct gtcttgaaga cacatcctgt gactatggct accacaggcg atttgcatgt 1020
   actgcacaca cacagggatt gagtcctggc tgttatgata cctatggtgc agacatagac 1080
   tgccagtgga ttgatattac agatgtaaaa cctggaaact atatcctaaa ggtcagtgta 1140
   aaccccagct acctggttcc tgaatctgac tataccaaca atgttgtgcg ctgtgacatt 1200
   cgctacacag gacatcatgc gtatgcctca ggctgcacaa tttcaccgta ttag 1254
<210> 8
   <211> 417
   <212> PRT
   <213> Homo sapiens
<400> 8
<210> 9
   <211> 752
   <212> PRT
   <213> Homo sapiens
<400> 9
   Met Arg Pro Val Ser Val Trp Gln Trp Ser Pro Trp Gly Leu Leu Leu
   1               5                   10                  15
   Cys Leu Leu Cys Ser Ser Cys Leu Gly Ser Pro Ser Pro Ser Thr Gly
               20                  25                  30
   Pro Glu Lys Lys Ala Gly Ser Gln Gly Leu Arg Phe Arg Leu Ala Gly
           35                  40                  45
   Phe Pro Arg Lys Pro Tyr Glu Gly Arg Val Glu Ile Gln Arg Ala Gly
       50                  55                  60
   Glu Trp Gly Thr Ile Cys Asp Asp Asp Phe Thr Leu Gln Ala Ala His
   65                  70                  75                  80
   Ile Leu Cys Arg Glu Leu Gly Phe Thr Glu Ala Thr Gly Trp Thr His
                   85                  90                  95
   Ser Ala Lys Tyr Gly Pro Gly Thr Gly Arg Ile Trp Leu Asp Asn Leu
               100                 105                 110
   Ser Cys Ser Gly Thr Glu Gln Ser Val Thr Glu Cys Ala Ser Arg Gly
           115                 120                 125
   Trp Gly Asn Ser Asp Cys Thr His Asp Glu Asp Ala Gly Val Ile Cys
       130                 135                 140
   Lys Asp Gln Arg Leu Pro Gly Phe Ser Asp Ser Asn Val Ile Glu Val
   145                 150                 155                 160
   Glu His His Leu Gln Val Glu Glu Val Arg Ile Arg Pro Ala Val Gly
                   165                 170                 175
   Trp Gly Arg Arg Pro Leu Pro Val Thr Glu Gly Leu Val Glu Val Arg
               180                 185                 190
   Leu Pro Asp Gly Trp Ser Gln Val Cys Asp Lys Gly Trp Ser Ala His
           195                 200                 205
   Asn Ser His Val Val Cys Gly Met Leu Gly Phe Pro Ser Glu Lys Arg
       210                 215                 220
   Val Asn Ala Ala Phe Tyr Arg Leu Leu Ala Gln Arg Gln Gln His Ser
   225                 230                 235                 240
   Phe Gly Leu His Gly Val Ala Cys Val Gly Thr Glu Ala His Leu Ser
                   245                 250                 255
   Leu Cys Ser Leu Glu Phe Tyr Arg Ala Asn Asp Thr Ala Arg Cys Pro
               260                 265                 270
   Gly Gly Gly Pro Ala Val Val Ser Cys Val Pro Gly Pro Val Tyr Ala
   275 280 285
   Ala Ser Ser Gly Gln Lys Lys Gln Gln Gln Ser Lys Pro Gln Gly Glu
   290 295 300
   Ala Arg Val Arg Leu Lys Gly Gly Ala His Pro Gly Glu Gly Arg Val
   305 310 315 320
   Glu Val Leu Lys Ala Ser Thr Trp Gly Thr Val Cys Asp Arg Lys Trp
   325 330 335
   Asp Leu His Ala Ala Ser Val Val Cys Arg Glu Leu Gly Phe Gly Ser
   340 345 350
   Ala Arg Glu Ala Leu Ser Gly Ala Arg Met Gly Gln Gly Met Gly Ala
   355 360 365
   Ile His Leu Ser Glu Val Arg Cys Ser Gly Gln Glu Leu Ser Leu Trp
   370 375 380
   Lys Cys Pro His Lys Asn Ile Thr Ala Glu Asp Cys Ser His Ser Gln
   385 390 395 400
   Asp Ala Gly Val Arg Cys Asn Leu Pro Tyr Thr Gly Ala Glu Thr Arg
   405 410 415
   Ile Arg Leu Ser Gly Gly Arg Ser Gln His Glu Gly Arg Val Glu Val
   420 425 430
   Gln Ile Gly Gly Pro Gly Pro Leu Arg Trp Gly Leu Ile Cys Gly Asp
   435 440 445
   Asp Trp Gly Thr Leu Glu Ala Met Val Ala Cys Arg Gln Leu Gly Leu
   450 455 460
   Gly Tyr Ala Asn His Gly Leu Gln Glu Thr Trp Tyr Trp Asp Ser Gly
   465 470 475 480
   Asn Ile Thr Glu Val Val Met Ser Gly Val Arg Cys Thr Gly Thr Glu
   485 490 495
   Leu Ser Leu Asp Gln Cys Ala His His Gly Thr His Ile Thr Cys Lys
   500 505 510
   Arg Thr Gly Thr Arg Phe Thr Ala Gly Val Ile Cys Ser Glu Thr Ala
   515 520 525
   Ser Asp Leu Leu Leu His Ser Ala Leu Val Gln Glu Thr Ala Tyr Ile
       530                 535                 540
   Glu Asp Arg Pro Leu His Met Leu Tyr Cys Ala Ala Glu Glu Asn Cys
   545                 550                 555                 560
   Leu Ala Ser Ser Ala Arg Ser Ala Asn Trp Pro Tyr Gly His Arg Arg
                   565                 570                 575
   Leu Leu Arg Phe Ser Ser Gln Ile His Asn Leu Gly Arg Ala Asp Phe
               580                 585                 590
   Arg Pro Lys Ala Gly Arg His Ser Trp Val Trp His Glu Cys His Gly
           595                 600                 605
   His Tyr His Ser Met Asp Ile Phe Thr His Tyr Asp Ile Leu Thr Pro
       610                 615                 620
   Asn Gly Thr Lys Val Ala Glu Gly His Lys Ala Ser Phe Cys Leu Glu
   625                 630                 635                 640
   Asp Thr Glu Cys Gln Glu Asp Val Ser Lys Arg Tyr Glu Cys Ala Asn
                   645                 650                 655
   Phe Gly Glu Gln Gly Ile Thr Val Gly Cys Trp Asp Leu Tyr Arg His
               660                 665                 670
   Asp Ile Asp Cys Gln Trp Ile Asp Ile Thr Asp Val Lys Pro Gly Asn
           675                 680                 685
   Tyr Ile Leu Gln Val Val Ile Asn Pro Asn Phe Glu Val Ala Glu Ser
       690                 695                 700
   Asp Phe Thr Asn Asn Ala Met Lys Cys Asn Cys Lys Tyr Asp Gly His
   705                 710                 715                 720
   Arg Ile Trp Val His Asn Cys His Ile Gly Asp Ala Phe Ser Glu Glu
                   725                 730                 735
   Ala Asn Arg Arg Phe Glu Arg Tyr Pro Gly Gln Thr Ser Asn Gln Ile
               740                 745                 750